(19) Europäisches Patentamt
European Patent Office
Office européen des brevets

(11) **EP 4 269 084 A1**

(12) **EUROPÄISCHE PATENTANMELDUNG**

(43) Veröffentlichungstag:
**01.11.2023   Patentblatt 2023/44**

(21) Anmeldenummer: **23154975.9**

(22) Anmeldetag: **03.02.2023**

(51) Internationale Patentklassifikation (IPC):
**B31D 5/00** (2017.01)    **A61L 9/00** (2006.01)
**A61L 9/12** (2006.01)    **B32B 1/00** (2006.01)
**B32B 3/26** (2006.01)    **D21H 21/14** (2006.01)

(52) Gemeinsame Patentklassifikation (CPC):
**B32B 3/26; A61L 9/12; A61L 9/127; B32B 1/00; D21H 21/14**

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**KH MA MD TN**

(30) Priorität: **03.02.2022   DE 202022100639 U**
**03.02.2022   LU 501389**

(71) Anmelder: **Mantz GmbH**
**42651 Solingen (DE)**

(72) Erfinder: **SEEFELD, Jörg**
**42651 Solingen (DE)**

(74) Vertreter: **Kudla, Christof**
**Klemens-Horn-Straße 22**
**42655 Solingen (DE)**

Bemerkungen:
Der Patentanspruch 15 (Anspruchsnummer 8 doppelt vergeben) gilt als fallen gelassen, da die entsprechende Anspruchsgebühr nicht entrichtet wurde (R. 45(3) EPÜ).

(54) **VERWENDUNG EINES MEHRSCHICHTIGEN PAPIERDIFFUSIONSSTABES ZUR ABGABE FLÜCHTIGER STOFFE IN DIE UMGEBUNG UND DAS GERÄT SELBST**

(57)    Die vorliegende Erfindung betrifft eine Verwendung eines Diffusionsstäbchens zum Freisetzen einer einen Wirkstoff enthaltenen flüssigen Zusammensetzung, wobei das Diffusionsstäbchen durch Aufrollen von Papier hergestellt wurde. Die vorliegende Erfindung betrifft zudem eine Vorrichtung zum Freisetzen eines Wirkstoffes in die Umgebung, bestehend aus oder umfassend a) eine flüssige Zusammensetzung bestehend aus oder umfassend a1) ein oder mehrere Lösungsmittel und a2) ein oder mehrere Wirkstoffe, b) mindestens ein erfindungsgemäßes Diffusionsstäbchen und c) einen Behälter, der zumindest eine Behälteröffnung aufweist und die flüssige Zusammensetzung umfasst. Die vorliegende Erfindung betrifft auch entsprechende Diffusionsstäbchen.

Fig. 5

EP 4 269 084 A1

**Beschreibung**

Die vorliegende Erfindung betrifft eine Verwendung eines Diffusionsstäbchens zum Freisetzen einer einen Wirkstoff enthaltenen flüssigen Zusammensetzung, wobei das Diffusionsstäbchen durch Aufrollen von Papier hergestellt wurde. Die vorliegende Erfindung betrifft zudem eine Vorrichtung zum Freisetzen eines Wirkstoffes in die Umgebung, bestehend aus oder umfassend a) eine flüssige Zusammensetzung bestehend aus oder umfassend a1) ein oder mehrere Lösungsmittel und a2) ein oder mehrere Wirkstoffe, b) mindestens ein erfindungsgemäßes Diffusionsstäbchen und c) einen Behälter, der zumindest eine Behälteröffnung aufweist und die flüssige Zusammensetzung umfasst. Die vorliegende Erfindung betrifft auch entsprechende Diffusionsstäbchen.

Zur Beduftung und Erfrischung von Räumen werden neben Versprühsystemen häufig auch Verdunstungssysteme verwendet. Diese Verdunstungssysteme haben im Vergleich mit Versprühsystemen den Vorteil, dass sie einen über die gesamte Laufzeit annähernd konstanten Duft erzeugen, während bei Versprühsystemen lediglich ein intensiver Duft nach jeweils einem Sprühstoß erzeugt wird, der allmählich abnimmt.

Verdampfungssysteme bestehen in der Regel aus einem Behälter, der einen in einem Lösungsmittel gelösten Duftstoff umfasst und ein in dem Behälter enthaltenden Diffusionsstäbchen. Die Diffusionsstäbchen sind dabei so ausgebildet, dass sie aufgrund von Kapillarkräften die organischen Lösungsmittel und den in dem Lösungsmittel gelösten Duftstoff aufnehmen und entlang des Diffusionsstäbchens aus dem Behälter befördern. An der Oberfläche der Diffusionsstäbchen verdampft das organische Lösungsmittel samt Duftstoff anschließend und der Duftstoff (nebst Lösungsmittel) wird in die Umgebung abgegeben.

Die im Stand der Technik beschriebenen Verdampfungssysteme weisen üblicherweise den Nachteil auf, dass Diffusionsstäbchen aus Kunststoff eingesetzt werden, die nicht verrotten, sofern sie versehentlich in die Umwelt gelangen. Zudem werden Kunststoffe überwiegend aus erdölbasierten Ausgangsubstanzen hergestellt und weisen daher eine nachteilige Öko- und $CO_2$-Bilanz auf. Als biologische Alternative zu Kunststoffdiffusionsstäbchen wird häufig Rattan eingesetzt. Rattan hat allerdings den Nachteil, dass es nicht mit allen Lösungsmitteln kompatibel ist. So quillt Rattan beispielsweise bei der Verwendung von Wasser als Lösungsmittel auf und die Diffusionswirkung des Rattans nimmt stark ab.

In der US 2011/0262377 A1 wird die Verwendung von Artikeln beschrieben, die aus einer Matrix bestehen, bei der es sich um aufgerolltes Papier handeln kann. Diese Matrix wird mit einem Duftstoff oder einer Duftstoffmischung getränkt. Im Anschluss kann der Artikel den Duftstoff über mehrere Tage freisetzen, wobei der Duftstoff an der Oberfläche des Artikels verdunstet und eine Diffusion vom Inneren des Artikels an die Oberfläche erfolgt. Die Verwendung des Artikels als Diffusionsstäbchen wird nicht beschrieben, da keine Förderung des Duftstoffes entlang des Artikels aus einem Behälter erfolgt.

In der US 2021/0205489 A1 wird die Verwendung eines Duftartikels beschrieben, der durch Aufrollen aus einem perforierten Papier hergestellt wurde und mit einer Duftstoffmischung getränkt ist. Zudem weist der Duftartikel an der Außenseite eine für die Duftstoffmischung durchlässige Polymerfolie auf. Die Duftstoffmischung verdunstet an der Oberfläche des Artikels. Die Verwendung des Artikels als Diffusionsstäbchen wird nicht beschrieben, da keine Förderung der Duftstoffmischung entlang des Duftartikels aus einem Behälter erfolgt.

In der DE 202021100549U1 wird die Verwendung von Diffusionsstäbchen beschrieben, die Fasern pflanzlicher Herkunft umfassen oder aus Fasern pflanzlicher Herkunft bestehen. Die einen Enden der Diffusionsstäbchen sind in eine einen Wirkstoff enthaltende Zusammensetzung getaucht, während sich die anderen Enden der Diffusionsstäbchen aus dem Behälter, der die Zusammensetzung enthält, erstrecken. Die Diffusionsstäbchen werden nicht durch Aufrollen von Papier hergestellt.

Aufgabe der vorliegenden Erfindung war es, eine Diffusionsstäbchen zur Verfügung zu stellen, dass

a) eine hohe Diffusionswirkung über die gesamte Laufzeit aufweist,

b) mit unterschiedlichen Lösungsmitteln kompatibel ist und hierbei insbesondere auch mit Wasser als Lösungsmittel eingesetzt werden kann und

c) biologisch abbaubar ist.

Idealerweise sollte das Diffusionsstächen ohne Ausgangssubstanzen aus fossilen Quellen herstellbar sein und eine verbesserte Öko- und $CO_2$-Bilanz aufweisen.

Diese Aufgaben wurde gelöst durch die Verwendung eines Diffusionsstäbchens (1) in (vorzugsweise erfindungsgemäßen) Vorrichtungen (2) zum Freisetzen einer einen Wirkstoff enthaltenen flüssigen Zusammensetzung (3), wobei das Diffusionsstäbchen (1) durch Aufrollen von Papier (4) hergestellt wurde.

Eigene Untersuchungen haben überraschenderweise gezeigt, dass Diffusionsstäbchen (1), die durch Aufrollen von Papier (4) hergestellt wurden, hervorragende Eigenschaften bezüglich des Transportes von flüssigen Wirkstoffzusammensetzungen aufweisen. Es hat sich gezeigt, dass die Aufnahme und Freisetzung der Wirkstoffzusammensetzung über die gesamte Laufzeit von üblichen Verdampfungssystemen konstant bleibt. Hierbei hat es sich zudem überraschenderweise gezeigt, dass die Diffusionsstäbchen mit gängigen, in der Duftstoffindustrie verwendeten, Lösungsmittel und sogar mit Wasser kompatibel sind. Insbesondere die Kompatibilität mit Wasser ist für den Fachmann überraschend, da er davon ausgehen würde, dass Diffusionsstäbchen, die durch Aufrollen von Papier hergestellt wurden, bei längerem Kontakt mit Wasser erweichen und sich zersetzen, sodass keine weitere Aufnahme und Freisetzung der Wirkstoffzusammensetzung möglich ist.

Verglichen mit Diffusionsstäbchen die aus Fasern pflanzlicher Herkunft bestehen oder diese umfassen und nicht durch Aufrollen von Papier hergestellt wurden (beispielsweise durch Extrudieren oder mittels Fasergussverfahren) haben sich bei erfindungsgemäß verwendeten Diffusionsstäbchen folgende Vorteile gezeigt.

- Verbesserte Haltbarkeit der Diffusionsstäbchen während des Transportes, da die Diffusionsstäbchen nicht dazu neigen aufzuweichen (insbesondere mit wasserhaltigen flüssigen Wirkstoffzusammensetzungen) und zu knicken. Die Integrität des Diffusionsstäbchens bleibt während der gesamten Nutzungsdauer intakt.
- Verbesserter Transport der einen Wirkstoff enthaltenden flüssigen Zusammensetzung entlang der Längsachse des Diffusionsstäbchens.
- Verbesserte Beständigkeit gegenüber Wasser und mechanischer Einwirkung (insbesondere von bereits mit flüssiger Zusammensetzung beladener Diffusionsstäbchen).

Eine Verwendung ist erfindungsgemäß bevorzugt, wobei das Diffusionsstäbchen teilweise in die flüssige Zusammensetzung eintaucht.

Eine Verwendung ist erfindungsgemäß bevorzugt in einem Verdampfungssystem, das aus einem Behälter besteht, der die einen Wirkstoff enthaltene flüssigen Zusammensetzung und das Diffusionsstäbchen umfasst, wobei das Diffusionsstäbchen aufgrund von Kapillarkräften die flüssige Zusammensetzung aufnimmt und entlang des Diffusionsstäbchens aus dem Behälter befördert und anschließend die flüssige Zusammensetzung an der Oberfläche der Diffusionsstäbchen verdampft wird, wodurch der Wirkstoff (nebst weiteren Bestandteilen) in die Umgebung abgegeben wird.

Eine Verwendung ist erfindungsgemäß besonders bevorzugt, zum Transport der einen Wirkstoff enthaltenen flüssigen Zusammensetzung aus einem Behälter. Hierbei erfolgt der Transport vorzugsweise durch Kapillarkräfte, während ein Teil des Diffusionsstäbchen in dem Behälter eingeführt ist und ein Teil des Diffusionsstäbchens sich aus dem Behälter erstreckt.

Eine Verwendung ist erfindungsgemäß besonders bevorzugt, wenn das Diffusionsstäbchen an einem aufnehmenden Endbereich des Diffusionsstäbchens in die flüssige Zusammensetzung (3) eintaucht, um die flüssige Zusammensetzung aufzunehmen, und die flüssige Zusammensetzung an einem abgebenden Endbereich des Diffusionsstäbchens teilweise oder vollständig in die Umgebung abgegeben wird. Die Diffusionsstäbchen nehmen die flüssige Zusammensetzung an dem aufnehmenden Endbereich auf und befördern diese entlang des Diffusionsstäbchens bis zum abgebenden Endbereich, wo sie die flüssige Zusammensetzung vollständig oder teilweise in die Umgebung abgeben. Üblicherweise und erfindungsgemäß bevorzugt befindet sich die flüssige Zusammensetzung dabei in einem Behälter (9), der zumindest eine Behälteröffnung (10) aufweist, und die Diffusionsstäbchen erstrecken sich durch die Behälteröffnung (10) aus dem Behälter (9).

Es ist erfindungsgemäß besonders bevorzugt, wenn die Diffusionsstäbchen lotrecht (als auch senkrecht oder vertikal bezeichnet) oder überwiegend (bevorzugt max. 45° Abweichung zum Lot, vorzugsweise max. zur 30° Abweichung zum Lot, weiter bevorzugt max. 25° Abweichung zum Lot) lotrecht angeordnet sind. Die flüssige Zusammensetzung wird dabei entgegen der Schwerkraft vom aufnehmenden Endbereich des Diffusionsstäbchens bis zum abgebenden Endbereich des Diffusionsstäbchens transportiert.

Der Transport der flüssigen Zusammensetzung erfolgt dabei überwiegend entlang der Diffusionsstäbchen, wobei die flüssige Zusammensetzung an dem Endbereich die flüssige Zusammensetzung aufnimmt an dem Sie in die flüssige Zusammensetzung eintaucht und die flüssige Zusammensetzung an dem anderen Endbereich des Diffusionsstäbchens in die Umgebung abgibt.

Üblicherweise entspricht der Teil des Diffusionsstäbchens dem aufnehmenden Endbereich des Diffusionsstäbchens, der in die flüssige Zusammensetzung eintaucht, während der abgebenden Endbereich des Diffusionsstäbchens dem Teil des Diffusionsstäbchens entspricht, der nicht in die flüssige Zusammensetzung eintaucht.

Eine Verwendung ist erfindungsgemäß besonders bevorzugt, wobei das Papier keine Perforationen, Löcher oder Ausstanzungen aufweist. Eigene Untersuchungen haben gezeigt, dass Diffusionsstäbchen für den Transport entlang der Längsachse nicht geeignet sind bzw. schlechtere Transporteigenschaften aufweisen, wenn das eingesetzte Papier Perforationen, Ausstanzungen oder Löcher aufweist, wie dies beispielsweise in der US 2021/0205489 A1 beschrieben wird. Zudem weisen Diffusionsstäbchen, die durch Aufrollen von perforiertem Papier hergestellt wurden, eine geringere mechanische Beständigkeit auf, wenn sie mit einer flüssigen Zusammensetzung beladen sind.

Eine Verwendung ist erfindungsgemäß besonders bevorzugt, wobei das Paper, aus dem durch Aufrollen ein Diffusionsstäbchen hergestellt wurde, eine gleichmäßige Dicke aufweist.

Es hat sich überraschenderweise gezeigt, dass bei der erfindungsgemäßen Verwendung die Diffusionsstäbchen nicht durch Verschmutzungen, die in den Duftölen enthalten sein können, verstopfen, sondern über die gesamte Lebenszeit, d.h. bis die flüssige Zusammensetzung (üblicherweise 25 bis 250 mL, teilweise sogar bis 500 mL) aufgebraucht ist, funktionieren. Auch hat es sich überraschenderweise gezeigt, dass ein Wenden der erfindungsgemäß verwendeten Diffusionsstäbchen nicht notwendig ist. Bei Diffusionsstäbchen, die aus Bambus oder anderen Gräsern oder Hölzern bestehen, ist es häufig notwendig, dass die Diffusionsstäbchen aus dem Behälter genommen werden, gedreht werden und erneute in den Behälter gesteckt werden, damit das Ende der Diffusionsstäbchen, dass sich mit dem Duftstoff vollgesogen hat, den Duftstoff an die Umgebung außerhalb des Behälters abgeben kann.

Eine Verwendung ist erfindungsgemäß besonders bevorzugt, wobei das Diffusionsstäbchen keine Kunststoffe umfasst. Im Rahmen der vorliegenden Erfindung werden Werkstoffe als Kunststoffe bezeichnet, die hauptsächlich aus Makromolekülen bestehen. Stärke, Cellulose, modifizierter Cellulose und Lignine werden im Rahmen der vorliegenden Erfindung nicht als Kunststoffe bezeichnet.

Sofern Kunststoffe in erfindungsgemäß verwendeten Diffusionsstäbchen eingesetzt werden, ist es bevorzugt, wenn es sich um biologisch abbaubare Kunststoffe handelt. Unter biologisch abbaubaren Kunststoffen versteht man Kunststoffe, die durch Mikroorganismen wie Pilze oder Bakterien, mittels Enzymen unter bestimmten Bedingungen zersetzt werden können. Vorzugsweise sind die biologisch abbaubaren Kunststoffe abbaubar nach der Norm EN 14995.

Da die Diffusionsstäbchen vollständig oder überwiegend aus Papier bestehen, hat es sich überraschenderweise auch gezeigt, dass sie biologisch abbaubar sind und sogar dem Kompost oder Papierrecycling zugeführt werden können.

Im Vergleich mit Diffusionsstäbchen, die nicht durch Aufrollen von Papier hergestellt wurden, beispielsweise durch Extrusion von Pulpe, zeigen Diffusionsstäbchen, die durch Aufrollen von Papier hergestellt wurden, eine verbesserte Diffusionsleistung. Ohne sich auf eine bestimmte Theorie festlegen zu wollen, wird davon ausgegangen, dass aufgrund der feinen Zwischenschichten, die sich zwischen zwei Papierwindungen ausbilden, eine verbesserte Kapillarwirkung erreicht werden kann. Bei durch Aufrollen von Papier hergestellten Diffusionsstäbchen erfolgt der Transport sowohl aufgrund der Kapillarität innerhalb des Papiers als auch aufgrund der zwischen den Papierschichten vorliegenden Räume, die als Spalten angesehen werden können, und eine eigene Kapillarität begründen können.

Diese Annahme wird dadurch gestützt, dass abhängig von der eingestellten Festigkeit der Wicklung der Diffusionsstäbchen auch bei identischem Papier als Ausgangsstoff der Diffusionsstäbchen unterschiedliche Diffusionseigenschaften eingestellt werden können. So haben eigene Untersuchungen gezeigt, dass bei einer losen Wicklung des Papiers polare Flüssigkeiten besser transportiert werden als bei einer festen Wicklung des Papiers. Die Festigkeit der Wicklung lässt sich während des Herstellungsprozesses der Diffusionsstäbchen durch den Zusammenpressdruck steuern. Die Festigkeit der Wicklung hat eine Auswirkung auf die Dichte der hergestellten Diffusionsstäbchen und je höher die Festigkeit der Wicklung ist, desto größer ist die Dichte. Die Dichte der Diffusionsstäbchen ist somit ein Maß für den während des Herstellungsprozess verwendeten Zusammenpressdruck. Bevorzugt weisen die erfindungsgemäß verwendeten Diffusionsstäbchen eine Dichte im Bereich von 0,5 bis 1,4 g/cm$^3$ auf, vorzugsweise im Bereich von 0,6 bis 1,3 g/cm$^3$, weiter bevorzugt im Bereich von 0,7 bis 1,1 g/cm$^3$.

Bei Diffusionsstäbchen mit einer Dichte von größer 1,4 g/cm$^3$ ist die Dichte des Diffusionsstäbchens so groß, dass kaum noch Kapillaritäten innerhalb des Papiers festgestellt werden können und zudem sind die einzelnen Papierschichten so fest aneinandergedrückt, dass auch zwischen den Papierschichten keine ausreichenden Kapillaren ausgebildet werden. Bei Diffusionsstäbchen mit einer Dichte von kleiner 0,5 g/cm$^3$ weisen die Diffusionsstäbchen häufig keine ausreichende Stabilität auf.

Besonders bevorzugt sind Diffusionsstäbchen mit einer Dichte im Bereich von 0,5 bis 0,9 g/cm$^3$ auf, vorzugsweise im Bereich von 0,6 bis 0,9 g/cm$^3$, weiter bevorzugt im Bereich von 0,7 bis 0,85 g/cm$^3$. Diffusionsstäbchen mit einer Dichte von unter 0,9 g/cm$^3$ können durch eine lockere Wicklung erhalten werden und zeichnen sich durch eine besonders gute Diffusions- und Transporteigenschaft von wasserhaltigen Zusammensetzungen aus.

In einigen Ausgestaltungen der vorliegenden Erfindung sind auch Diffusionsstäbchen mit einer Dichte im Bereich von größer 0,9 bis kleiner gleich 1,5 g/cm$^3$ bevorzugt, vorzugsweise im Bereich von größer 0,9 bis kleiner gleich 1,3 g/cm$^3$, weiter bevorzugt im Bereich von größer 0,9 bis kleiner gleich 1,0 g/cm$^3$. Diffusionsstäbchen mit einer Dichte von über 0,9 g/cm$^3$ können durch eine feste Wicklung erhalten werden und zeichnen sich durch eine besonders hohe Festigkeit bei guten Diffusions- und Transporteigenschaft aus.

Es ist bevorzugt, dass die erfindungsgemäß verwendeten Diffusionsstäbchen als Zylinder, besonders bevorzugt als senkrechter Kreiszylinder, ausgestaltet sind und somit einen Durchmesser (D) des Zylinders aufweisen und eine Länge (L) (bei Zylindern wird die Länge häufig auch als Höhe bezeichnet). Entlang Ihrer Länge weisen erfindungsgemäß verwendete Diffusionsstäbchen dabei eine Achse (A) auf, bei der es sich um die Längsachse handelt.

In einer Ausgestaltung der erfindungsgemäßen Verwendung ist ein Ende oder sind beide Enden des Diffusionsstäbchens schräg abgeschnitten. Durch ein schräges Abschneiden kann die Aufnahme des Lösungsmittels verbessert werden, da die Fläche an dem oder den Enden höher ist, als wenn das oder die Enden senkrecht zur Achse (A) abgeschnitten wären.

Bei der Berechnung der Dichte der Diffusionsstäbchen wird die Seele, sofern diese vorhanden ist, nicht berücksichtigt. Lediglich die Dichte des Mantels wird betrachtet. Das Volumen des Diffusionsstäbchens kann mit der Formel

$$V = \pi \left(\frac{D}{2}\right)^2 L$$

berechnet werden, sofern das Diffusionsstäbchen keine Seele aufweist, oder mit der Formel

$$V = \pi \left(\left(\frac{D}{2}\right)^2 - \left(\frac{d}{2}\right)^2\right) L$$

berechnet werden, sofern das Diffusionsstäbchen eine Seele aufweist, wobei V das Volumen, D der Durchmesser des Diffusionsstäbchens, d der Durchmesser der Seele und L die Länge des Diffusionsstäbchens ist. Diese Formeln zu Berechnung des Volumens eines Zylinders bzw. Hohlzylinder sind dem Fachmann bekannt. Aus Gewicht und Volumen des Diffusionsstäbchens kann der Fachmann die Dichte berechnen. Sofern das Diffusionsstäbchen eine abweichende Geometrie aufweisen sollte, beispielsweise weil die Enden Schräg abgeschnitten sind, kann der Fachmann das Volumen über einfache geometrische Formeln errechnen. Um das Gewicht möglichst genau zu bestimmen ist es ratsam, eine höhere Zahl an Diffusionsstäbchen zu messen und anschließend das gemessene Gewicht durch die Anzahl der Stäbchen zu dividieren.

Durch Einstellung der Wicklungsfestigkeit lässt sich somit der Raum bzw. Abstand zwischen zwei Papierschichten einstellen, sodass ein Anpassen des Diffusionsstäbchens an unterschiedliche Zusammensetzungen und an gewünschte Verdampfungsgeschwindigkeiten erreicht werden kann.

Eine Verwendung ist erfindungsgemäß bevorzugt, bei der die Diffusionsstäbchen (1) eine hohle Seele (5) entlang ihrer Achse (A) aufweisen. In dieser Ausgestaltung handelt es sich bei dem Diffusionsstäbchen um eine Hohlzylinder mit einem (Außen)Durchmesser (D), einem Durchmesser der Seele (Innendurchmesser (d)) und einer Wanddicke (W).

Überraschenderweise hat es sich gezeigt, dass Diffusionsstäbchen, die eine Seele entlang ihrer Achse aufweisen eine höhere Verdunstungsrate aufweisen. Ohne sich dabei auf eine Theorie festlegen zu wollen, wird davon ausgegangen, dass die Seele als Kapillare wirkt und den Transport der Zusammensetzung weiter verbessert. Wie allgemein bekannt ist die Kapillarwirkung einer Röhre vom Durchmesser der Kapillare abhängig. Eigene Untersuchungen haben dabei gezeigt, dass bei einem Durchmesser von weniger als 0,60 mm die Kapillarität so hoch ist, dass die gesamte Seele mit der Zusammensetzung gefüllt ist. Bei einem Durchmesser der Seele von mehr als 4,0 mm hingegen ist die Kapillarwirkung der Seele häufig nur gering ausgeprägt.

Diffusionsstäbchen mit einem Durchmesser der Seele von kleiner gleich 0,50 mm werden im Rahmen der vorliegenden Erfindung als Diffusionsstäbchen ohne Seele angesehen.

Üblicherweise ist der Querschnitt der Seele überwiegend rund ausgebildet, wobei der Querschnitt auch überwiegend oval ausgebildet sein kann. Sofern der Querschnitt der Seele nicht rund ausgebildet ist, handelt es sich bei dem Durchmesser der Seele um den durchschnittlichen Durchmesser, der aus großer Halbachse und kleiner Halbachse bestimmt wird.

Eigene Untersuchungen haben gezeigt, dass die Kapillarität besonders ausgeprägt ist, wenn die Polaritäten von Diffusionsstäbchen und Zusammensetzung aneinander angepasst sind. Eigene Untersuchungen haben dabei überraschenderweise ergeben, dass aufgrund der hohen Polarität von Papier wasserhaltige Zusammensetzungen eine besonders gute Kapillarität aufweisen.

Es ist erfindungsgemäß bevorzugt, wenn die Seele an den Enden der Diffusionsstäbchen offen ist und der Durchmesser an den Enden maximal 50 % geringer ist als der durchschnittliche Durchmesser der Seele entlang des Diffusionsstäbchens, vorzugsweise maximal 25 % geringer, weiter bevorzugt maximal 10 % geringer. Nach dem Zusammenrollen des Papiers zu einem Diffusionsstäbchen müssen die Diffusionsstäbchen üblicherweise auf Länge geschnitten werden und durch den Schnitt kann es vorkommen, dass der Durchmesser der Seele an den Enden geringer ist als entlang des Diffusionsstäbchens. Es ist erfindungsgemäß nicht bevorzugt und zu vermeiden, dass die Enden des Diffusionsstäbchen verschlossen sind, obwohl eine Seele innerhalb des Diffusionsstäbchens ausgebildet ist.

Eine Verwendung ist erfindungsgemäß bevorzugt, wobei die Seele einen Durchmesser von mindestens 0,50 mm aufweist, vorzugsweise mindestens 0,60 mm aufweist, weiter bevorzugt mindestens 0,70 mm aufweist.

Eine Verwendung ist erfindungsgemäß bevorzugt, wobei die Seele einen Durchmesser von maximal 3,5 mm aufweist, vorzugsweise maximal 2,5 mm, weiter bevorzugt maximal 2,0mm.

Eine Verwendung ist erfindungsgemäß bevorzugt, wobei die Seele einen Durchmesser im Bereich von 0,50 mm bis 3,5 mm aufweist, vorzugsweise im Bereich von 0,60 mm bis 3,0 mm aufweist, weiter bevorzugt im Bereich von 0,70 mm bis 2,0 mm aufweist.

Erfindungsgemäß können Diffusionsstäbchen mit unterschiedlichen Längen, z. B. im Bereich von 50 bis 360 mm, und Durchmesser, z. B. im Bereich von 0,5 bis 15 mm verwendet werden, wobei hiervon auch abwichen werden kann.

Längere Diffusionsstäbchen können auch eingesetzt werden, allerding haben eigene Untersuchungen gezeigt, dass häufig die Kapillarkräfte nicht ausreichend hoch sind, um die an der Oberfläche der Diffusionsstäbchen verdampfende Zusammensetzung nachzuliefern und somit keine Zusammensetzung an das obere Ende der Diffusionsstäbchen gelangt und dieses somit trocken bleibt.

Eine Verwendung ist erfindungsgemäß allerdings bevorzugt, wobei das Diffusionsstäbchen einen Durchmesser im Bereich von 1,0 bis 10,0 mm aufweist, vorzugsweise im Bereich von 2,5 bis 7,0 mm aufweist, besonders bevorzugt im Bereich von 3,0 bis 5,0 mm aufweist. Eine Verwendung ist erfindungsgemäß allerdings bevorzugt, wobei das Diffusionsstäbchen einen Durchmesser von weniger als 5,5 mm, vorzugsweise, weniger als 5,0 mm, weiter bevorzugt von weniger als 4,5 mm aufweist.

Eine Verwendung ist erfindungsgemäß zudem bevorzugt, wobei das Diffusionsstäbchen eine Länge im Bereich von 50 bis 300 mm aufweist, vorzugsweise im Bereich von 80 bis 260 mm aufweisen, besonders bevorzugt im Bereich von 90 bis 240 mm aufweisen, weiter bevorzugt im Bereich von 170 bis 240 mm aufweisen.

Eine Verwendung ist erfindungsgemäß bevorzugt, wobei das Diffusionsstäbchen eine Länge im Bereich von 160 bis 300 mm aufweisen und einen Durchmesser von weniger als 5,5 mm, vorzugsweise, weniger als 5,0 mm, weiter bevorzugt von weniger als 4,5 mm aufweist. Es hat sich gezeigt, dass Diffusionsstäbchen mit einer Länge von mehr als 160 mm und einem Durchmesser von weniger als 5,5 mm besonders gute Eigenschaften bezüglich des Transportes der flüssigen Zusammensetzung aufweisen und gute Eigenschaften bezüglich der Verdampfungsgeschwindigkeit aufweisen. Kürzere Diffusionsstäbchen haben meist eine zu geringe Verdampfungsoberfläche, während bei zu großen Durchmessern wiederrum eine zu hohe Verdampfungsoberfläche vorliegt. Optimale Ergebnisse werden erreicht, wenn das Diffusionsstäbchen eine Länge von mehr als 160 mm und einem Durchmesser von weniger als 5,5 mm aufweist.

Bei einer zu geringen Wanddicke ist die mechanische Beständigkeit der Diffusionsstäbchen häufig nicht ausreichend und auch die Transport- und Diffusionseigenschaften nehmen häufig ab.

Es versteht sich natürlich, dass bei Diffusionsstäbchen, die eine Seele aufweisen, die Durchmesser der Diffusionsstäbchen entsprechend an den Durchmesser der Seele angepasst wird. So muss beispielsweise bei einem Diffusionsstäbchen mit einem Seelendurchmesser von 1,8 mm der Durchmesser des Diffusionsstäbchens natürlich größer sein als 1,8 mm.

Eine Verwendung ist zudem erfindungsgemäß bevorzugt, wobei das Diffusionsstäbchen eine Wanddicke im Bereich von 0,4 bis 3,4 mm aufweist, vorzugsweise im Bereich von 0,5 bis 2,0, besonders bevorzugt im Bereich von 0,7 bis 2,5 mm.

Eine Verwendung ist erfindungsgemäß bevorzugt, wobei das Diffusionsstäbchen ein Verhältnis zwischen Länge und Durchmesser im Bereich von 10 : 1 bis 100 : 1 aufweist, vorzugsweise im Bereich von 20 : 1 bis 85 : 1, ganz besonders bevorzugt im Bereich von 50:1 bis 75:1.

Eine Verwendung ist erfindungsgemäß bevorzugt, wobei das Diffusionsstäbchen ein Verhältnis zwischen Wanddicke und Seelendurchmesser im Bereich von 30 : 1 bis 1 : 2 aufweist, vorzugsweise im Bereich von 20 : 1 bis 2 : 3, weiter bevorzugt im Bereich von 5 : 1 bis 1 : 1.

Eigene Untersuchungen haben gezeigt, dass in den oben aufgeführten Verhältnissen zwischen Wanddicke und Seelendurchmesser besonders gute Kapillareigenschaften und Stabilitäten der Diffusionsstäbchen erhalten werden können.

Eine Verwendung ist erfindungsgemäß bevorzugt, wobei die Wand des Diffusionsstäbchens aus 3 bis 55 Papierschichten besteht, vorzugsweise aus 5 bis 25 Papierschichten, weiter bevorzugt aus 6 bis 16 Papierschichten.

Wie weiter oben bereits ausgeführt, wird davon ausgegangen, dass sowohl die Kapillarität innerhalb der Papierschichten als auch die Kapillarität zwischen den Papierschichten einen Einfluss auf die Gesamtkapillarität und somit auf die Verdunstungsrate aufweist. Eigene Untersuchungen haben gezeigt, dass besonders gute Eigenschaften eingestellt werden könne, wenn die Wand des Diffusionsstäbchens mindestens 3 Papierschichten aufweist (dies entspricht zwei Trennschichten, die als Kapillaren dienen können). Der Kapillareffekt wird allerdings verbessert, wenn die Zahl der Papierschichten zunimmt. Optimale Eigenschaften wurden in eigenen Untersuchungen erzielt, wenn die Zahl der Papierschichten im Bereich von 6 bis 16 Papierschichten liegt. Bei mehr als 55 Papierschichten wird die Wanddicke der Diffusionsstäbchen zu groß und eine Verbesserung der Gesamtkapillarität mit Zunahme der Papierschichten erfolgt nicht oder nur im geringen Umfang.

Auch wenn die Diffusionsstäbchen keine Seele aufweisen, hat die Anzahl der Papierschichten die gleichen Auswirkungen. In diesem Fall wird formell davon ausgegangen, dass die Seele einen Durchmesser von 0 mm aufweist und die Wand der Hälfte des Durchmessers des Diffusionsstäbchens beträgt. Eine Verwendung ist daher erfindungsgemäß bevorzugt, wobei die Hälfte des Diffusionsstäbchens aus 3 bis 55 Papierschichten besteht, vorzugsweise aus 5 bis 25 Papierschichten, weiter bevorzugt aus 6 bis 16 Papierschichten.

Auch die Dicke bzw. Grammatur des für die Herstellung des Diffusionsstäbchens eingesetzten Papiers hat eine Auswirkung auf die Eigenschaften des resultierenden Diffusionsstäbchens. Bei dünnerem Papier können mehr Windungen auf eine bestimmte Dicke des Diffusionsstäbchens ausgebildet werden. Eigene Untersuchungen habe ergeben, dass es erfindungsgemäß bevorzugt ist, wenn

das Papier eine Dicke von mindestens 10 $\mu$m aufweist, vorzugsweise mindestens 40 $\mu$m, weiter bevorzugt mindesten 60 $\mu$m aufweist und/oder.

das Papier eine Dicke von maximal 400 μm aufweist, vorzugsweise maximal 300 μm, weiter bevorzugt maximal 150 μm aufweist.

Eine Verwendung ist somit erfindungsgemäß bevorzugt, wobei das Papier eine Dicke im Bereich von 10 μm bis 400 μm aufweist, vorzugsweise im Bereich von 40 μm bis 300 μm, weiter bevorzugt im Bereich von 60 μm bis 150 μm aufweist.

Es gibt unterschiedliche Methoden, wie das Papier zur Ausbildung eines Diffusionsstäbchens aufgerollt werden kann. Es ist erfindungsgemäß bevorzugt, wenn das Papier entlang des gesamten Diffusionsstäbchens aufgerollt wird und hierbei mehrere übereinanderliegende Lagen ausbildet. Das Blatt wird hierbei parallel zur gesamten Mantelfläche des Zylinders in mehreren Lagen gewickelt.

Zur Herstellung des Diffusionsstäbchens können unterschiedliche Papiermaterialien eingesetzt werden. Eigene Untersuchungen haben allerdings gezeigt, dass nicht alle Papiermaterialien gleich gute Eigenschaften aufweisen.

Es ist erfindungsgemäß bevorzugt, wenn das Papier aus Zellstofffasern besteht oder diese umfasst und es sich bei den Zellstofffasern um Nadelholz-, Laubholz-, Gras-, Strohzellstofffasern oder Mischungen daraus handelt.

Sofern die Zellstofffasern aus Nadelholz oder Laubholz hergestellt wurden, hat es sich als vorteilhaft erwiesen, wenn es sich bei den Zellstofffasern um Fasern aus Kiefern-, Birken, Pappeln, Fichten-, Buchen- oder Eukalyptusholz handelt.

In einer besonders bevorzugten Ausgestaltung der vorliegenden Erfindung handelt es sich den Zellstofffasern um Fasern aus Süßgrasfasern, vorzugsweise Zuckerrohrfasern, Weidengras oder Bambusfasern. Süßgrasfasern haben nicht nur den Vorteil, dass die Materialien schnell nachwachsen, sondern es hat sich in eigenen Untersuchungen gezeigt, dass die aus den Fasern hergestellten Papieren besonders gute Kapillareigenschaften aufweisen.

Es ist erfindungsgemäß bevorzugt, wenn das Papier als Zellstofffasern Frischfasern umfasst und der Massenanteil an Frischfasern größer gleich 80 % beträgt, bevorzugt größer gleich 90 %, besonders bevorzugt größer gleich 95 %, bezogen auf die Gesamtmasse der Zellstofffasern und bestimmt als lufttrockener Massenanteil (Lutro).

Es ist erfindungsgemäß bevorzugt, wenn sämtliche Zellstofffasern im Papier Frischfasern sind, d.h. wobei der Massenanteil an Frischfasern 100 % beträgt, bezogen auf die Gesamtmasse der Zellstofffasern und bestimmt als lufttrockener Massenanteil (Lutro).

Es ist erfindungsgemäß allerdings auch möglich, wenn das Diffusionsstäbchen als Zellstofffasern Recycling-Fasern umfasst und der Massenanteil an Recycling-Faser größer gleich 40 % beträgt, bevorzugt größer gleich 50 %, besonders bevorzugt größer gleich 60 %, bezogen auf die Gesamtmasse der Zellstofffasern und bestimmt als lufttrockener Massenanteil (Lutro).

Es ist erfindungsgemäß bevorzugt, wenn das Diffusionsstäbchen (1) zusätzlich Hilfsstoffe umfasst ausgewählt aus der Gruppe anorganische Füllstoffe, Bindemittel (vorzugsweise Stärke), Alaun und/oder Leim.

In der Papierherstellung ist es üblich, dass die hergestellten Papiere gebleicht werden, da die resultierenden gebleichten Papiere für den Konsumenten optisch ansprechender sind. Eigene Untersuchungen haben gezeigt, dass das Bleichen einen Einfluss auf die Kapillarwirkung des Papiers hat. Ohne sich auf eine bestimmte Theorie festlegen zu wollen, wird davon ausgegangen, dass das Bleichen einen Einfluss auf die Polarität des Papiers hat. Bei Verwendung von polaren Lösungsmitteln für die Wirkstoffzusammensetzung und hierbei insbesondere Wasser hat die Änderung der Polarität des Papiers einen Einfluss auf die Kapillarität des Materials. Eigene Untersuchungen haben überraschenderweise gezeigt, dass gebleichte Papiere bzw. Diffusionsstäbchen, die durch Aufrollen von ungebleichtem Papier hergestellt wurden, verbesserte Verdunstungsraten aufweisen, als ansonsten gleiche Diffusionsstäbchen, bei denen ein ungebleichtes Papier eingesetzt wurde. Erfindungsgemäß bevorzugt sind daher Diffusionsstäbchen, die aus gebleichtem Papier hergestellt wurden.

Als Papier zur Herstellung der Diffusionsstäbchen können unterschiedliche Papiere eingesetzt werden. Üblicherweise wird ein ungestrichenes Papier bzw. Naturpapier eingesetzt, das vor dem Aufrollen zum Diffusionsstäbchen vollständig oder teilweise mit einem wasserhaltigem oder aus Wasser bestehendem Additiv benetzt wird. Neben Wasser kann das Additiv auch ein Bindemittel, vorzugsweise Stärke, umfassen.

In einigen Ausgestaltungen ist es allerdings vorteilhaft, wenn das eingesetzte Papier ein- oder beidseitig mit einer Beschichtung beschichtet ist. Die Beschichtung von Papieren ist dem Fachmann bekannt und entsprechend beschichtete Papiere werden häufig als gestrichene Papiere bezeichnet.

Eine Verwendung ist erfindungsgemäß bevorzugt, wobei die Beschichtung ein anorganisches Pigment enthält, ausgewählt aus der Gruppe bestehend aus kalziniertem Kaolin, Kaolin, Kaolinit, Magnesiumsilikathydrat, Siliziumoxid, Bentonit, Calziumcarbonat, Aluminiumhydroxid, Aluminiumoxid und Böhmit.

Die Pigmente können eine positive Auswirkung auf die Kapillarität des Papiers haben und diese kann durch die Menge und die Auswahl der Pigmente eingestellt werden.

Eine Verwendung ist erfindungsgemäß bevorzugt, wobei die Beschichtung ein Bindemittel enthält, ausgewählt aus der Gruppe bestehend aus Stärke, insbesondere Kartoffelstärke, Maisstärke und Weizenstärke, Polyvinylalkohol, modifizierter Cellulose, carboxylgruppen-modifiziertem Polyvinylalkohol, Ethylen-Vinylalkohol-Copolymer, EthylenVinylacetat-Copolymer, silanolgruppen-modifiziertem Polyvinylalkohol, diacetonmodifiziertem Polyvinylalkohol, Acrylat-Copolymer, modifiziertem Polyethylenglycol, unmodifiziertem Polyethylenglycol, Styrol-Butadien-Latex, Styrol-Acrylat-Polymeren und Mischungen hieraus.

Besonders bevorzugt ist hierbei die Verwendung von Stärke als Bindemittel, insbesondere Kartoffelstärke, Maisstärke und Weizenstärke. Diese Bindemittel werden im Rahmen der vorliegenden Erfindung nicht als Kunststoffe angesehen.

Es ist erfindungsgemäß bevorzugt, wenn der Massenanteil an Zellstofffasern in dem Diffusionsstäbchen größer gleich 60,0 % beträgt, vorzugsweise größer gleich 70,0 % beträgt, besonders bevorzugt größer 75,0 % beträgt, bezogen auf die Gesamtmasse des Diffusionsstäbchen und bestimmt als lufttrockener Massenanteil (Lutro).

Ebenfalls ist es erfindungsgemäß bevorzugt, wenn der Massenanteil an Zellstofffasern in dem Diffusionsstäbchen kleiner gleich 95,0 % beträgt, vorzugsweise kleiner gleich 85 % beträgt, besonders bevorzugt kleiner gleich 80,0 % beträgt, bezogen auf die Gesamtmasse des Diffusionsstäbchen und bestimmt als lufttrockener Massenanteil (Lutro).

Es ist erfindungsgemäß bevorzugt, wenn der Massenanteil an Zellstofffasern in dem Diffusionsstäbchen im Bereich von 60,0 % bis 95,0 % liegt, vorzugsweise im Bereich von 70,0 % bis 85,0 % liegt, besonders bevorzugt im Bereich von 75,0 % bis 80,0 % liegt, bezogen auf die Gesamtmasse des Diffusionsstäbchen und bestimmt als lufttrockener Massenanteil (Lutro).

Der Wassergehalt bei lufttrockenen Diffusionsstäbchen liegt üblicherweise im Bereich von 1 bis 7 Gew.-%.

Es ist erfindungsgemäß bevorzugt, wenn das Papier, das zur Herstellung der Diffusionsstäbchen verwendet wird, ein Bindemittel umfasst, wobei es sich bei dem Bindemittel vorzugsweise um Stärke handelt. Bei der Stärke kann es sich um Stärke handeln ausgewählt aus der Gruppe bestehend aus natürlicher Stärke, anionischer Stärke, kationischer Stärke, peroxidierter Stärke, veretherter Stärke, veresterter Stärke, oxidierter Stärke, hydrolysierter Stärke, dextrinierter Stärke, hydroxyethylierter Stärke und acetylierter Stärke. Die Verwendung von natürlicher Stärke ist erfindungsgemäß besonders bevorzugt.

Hierbei ist es erfindungsgemäß bevorzugt, wenn der Massenanteil an Bindemittel im Bereich von 1,0 % bis 10,0 % liegt, vorzugsweise im Bereich von 2,0 % bis 8,0 % liegt, besonders bevorzugt im Bereich von 3,0 % bis 5,0 % liegt, bezogen auf die Gesamtmasse des Papiers und bestimmt als lufttrockenen Massenanteil (Lutro).

Um die Optik des Diffusionsstäbchen für den Konsumenten ansprechender zu gestalten, besteht die Möglichkeit, dass das Papier gefärbt und/oder bedruckt ist. Das Einfärben des Papiers kann beispielsweise in rosa, blau, grün, gelb oder durch Zusatz von anorganischen Pigmenten (s.o.) weiß erfolgen. Die Bedruckung der Papierstäbchen lässt sich mit unterschiedlichen Druckmethoden erreichen, die dem Fachmann bekannt sind. Es ist allerdings darauf zu achten, dass die eingesetzten Drucktinten bzw. Farbstoffe zum Einfärben des Papiers nicht oder nur geringfügig in den Lösungsmitteln der Wirkstoffzusammensetzung löslich sind.

In einigen Ausgestaltungen der vorliegenden Erfindung ist es vorteilhaft, wenn das Papier vor dem Aufrollen kalandriert wurde. Durch das Kalandrieren kann ein dichteres Papier mit einer höheren Oberflächenglätte erhalten werden. Die Glätte und Dichte des Papiers kann über die Linienlast des Kalanders eingestellt werden. Hierbei ist allerdings darauf zu achten, dass das Papier nicht zu glatt und zu dicht wird. Das Papier sollte bei einer Linienlast von weniger als 100kN/m (vorzugsweise weniger als 80 kN/m, weiter bevorzugt weniger als 50 kN/m) und einer Temperatur von maximal 100°C (vorzugsweise maximal 75 °C, weiter bevorzugt maximal 50 °C) kalandriert werden. In einigen Ausgestaltungen der vorliegenden Erfindung ist es allerdings vorteilhaft, wenn das Papier vor dem Aufrollen nicht kalandriert wurde. In diesem Fall ist die Oberfläche des Papiers rauer und das Papier nicht so dicht, wodurch die Kapillarität des Papiers verbessert werden kann.

Erfindungsgemäß ist eine Verwendung, bei der das Papier mit der Siebseite nach innen oder außen aufgerollt wird, bevorzugt ist die Siebseite nach innen angeordnet.

Erfindungsgemäß bevorzugt ist eine Verwendung, wobei das Diffusionsstäbchen keine Polymerfolie umfasst.

Eine Verwendung ist erfindungsgemäß bevorzugt, wobei das Papier vor dem Aufrollen mit einem Additiv beschichtet wurde, sodass das Additiv vorzugsweise zwischen den einzelnen Lagen des Diffusionsstäbchens angeordnet ist.

Hierbei ist es bevorzugt, wenn das Additiv Wasser und/oder ein Bindemittel umfasst oder aus Wasser gebildet ist. Besonders bevorzugt wird als Additiv eine Mischung aus Wasser und Stärke oder lediglich Wasser verwendet. Die Verwendung von lediglich Wasser ist vorteilhaft, wenn das Papier bereits ein- oder zweiseitig beschichtet ist, beispielsweise mit einem stärkehaltigen Strich. Durch das Auftragen von Wasser wird in diesem Fall der stärkehaltige Strich angelöst und dies führt nach dem Aufrollen des Papiers zu einem besseren Halt der einzelnen Schichten untereinander und einer erhöhten Stabilität des Diffusionsstäbchens. Der Auftrag des Additivs kann vollflächig oder nur an den Rändern des Papiers erfolgen. Vorzugsweise erfolgt der Auftrag des Additivs am Rand des Papiers, das die letzte Lage des Diffusionsstäbchens bildet. Hierdurch wir ein Abrollen des Papiers verhindert.

Eine Verwendung ist bevorzugt, bei der das Diffusionsstäbchen nach dem Aufrollen getrocknet wird.

Herstellungsverfahren zur Herstellung von Diffusionsstäbchen, die erfindungsgemäß verwendet werden können, werden beispielsweise in der US 2,357,846 oder der WO 2021/037920 A2 beschrieben.

Die einen Wirkstoff enthaltenen flüssigen Zusammensetzung umfassen neben einem, zwei oder mehr als zwei Wirkstoffen üblicherweise auch ein Lösungsmittel. Hierbei ist es bevorzugt, wenn es sich bei dem Lösungsmittel um Wasser handelt. Wasser hat den Vorteil, dass es günstig und absolut ungiftig ist.

Eine Verwendung ist ebenfalls bevorzugt, wobei die Zusammensetzung mindestens ein Lösungsmittel umfasst, ausgewählt aus der Gruppe bestehend aus Dipropylenglycolmethylether, Dipropylenglycolmethylether-acetat Diethylenglycolmonoethylether und Ethanol umfasst. Diese Lösungsmittel können auch mit Wasser kombiniert werden.

Hierbei ist es bevorzugt, wenn die einen Wirkstoff enthaltene flüssigen Zusammensetzung mindestens 5 Gew.-%, vorzugsweise mindestens 6 Gew.-%, besonders bevorzugt mindestens 8 Gew.-% dieser Lösungsmittel umfasst, jeweils bezogen auf das Gesamtgewicht der flüssigen Zusammensetzung.

Es ist ebenfalls bevorzugt, wenn die einen Wirkstoff enthaltene flüssigen Zusammensetzung 5 bis 90 Gew.-%, vorzugsweise 6 bis 70 Gew.-%, besonders bevorzugt 8 bis 65 Gew.-% dieser Lösungsmittel umfasst, jeweils bezogen auf das Gesamtgewicht der flüssigen Zusammensetzung.

Sofern die flüssige Zusammensetzung kein Wasser oder weniger als 1 Gew.-% Wasser umfasst ist es erfindungsgemäß bevorzugt, wenn die einen Wirkstoff enthaltene flüssigen Zusammensetzung mindestens 10 Gew.-%, vorzugsweise mindestens 15 Gew.-%, besonders bevorzugt mindestens 30 Gew.-% dieser Lösungsmittel umfasst, jeweils bezogen auf das Gesamtgewicht der flüssigen Zusammensetzung.

Es ist erfindungsgemäß bevorzugt, wenn die Zusammensetzung zusätzlich ein Tensid enthält. Insbesondere bei einem hohen Wasseranteil kann es notwendig werden, dass ein Tensid zugesetzt wird, um eine Emulsion aus Wirkstoff und Lösungsmittel herzustellen. Ob ein Tensid verwendet werden muss, hängt von der Löslichkeit des Wirkstoffes in dem Lösungsmittel (insbesondere Wasser) ab.

Es ist erfindungsgemäß bevorzugt, wenn die Zusammensetzung eine homogene Phase ausbildet.

Eigenen Untersuchungen haben ergeben, dass es erfindungsgemäß bevorzugt ist, wenn das Tensid einen nach Griffin berechneten HLB-Wert im Bereich von 3 bis 20 aufweist, vorzugsweise im Bereich von 6 bis 20, besonders bevorzugt im Bereich von 9 bis 20.

Erfindungsgemäß bevorzugt handelt es sich bei dem Tensid um Tenside ausgewählt aus der Gruppe bestehend aus nichtionischen Tensiden, anionischen Tensiden, kationischen Tensiden und amphotere Tenside.

Erfindungsgemäß besonders bevorzugt handelt es sich bei dem nichtionischen Tensid um eine Verbindung, die mehrere Alkohol-Gruppen aufweist, um eine Verbindung, die eine oder mehrere Ether-Gruppen aufweist, oder um eine Verbindung, die sowohl Alkohol-Gruppen als auch Ether-Gruppen aufweist (z. B. Ethoxylate).

Erfindungsgemäß besonders bevorzugt handelt es sich bei dem anionischen Tensid um eine Verbindung, die eine oder mehrere Carboxylat-Gruppen aufweist, um eine Verbindung, die eine oder mehrere Sulfonat-Gruppen aufweist, oder um eine Verbindung, die eine oder mehrere Sulfat-Gruppen aufweist.

Erfindungsgemäß besonders bevorzugt handelt es sich bei dem kationischen Tensid um eine Verbindung, die eine oder mehrere quartäre Ammonium-Gruppen aufweist.

Erfindungsgemäß besonders bevorzugt handelt es sich bei dem amphoteren Tensid um eine Verbindung, die sowohl Carboxylat-Gruppen als auch quartäre Ammonium-Gruppen aufweisen.

Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen.

Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Alkylethersulfate, Glyce- rinethersulfate, Fettsäureethersulfate, Hydroxymischethersulfate, Monoglycerid-(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N- Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate, Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisothionate, Alkylphosphate, Alkyletherphosphate, Alkylethercarboxylate, Alpha-Olefinsulfonate, insbesondere die Alkali- und Erdalkalimetallsalze, z. B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid- oder Propylenoxideinheiten, bevorzugt 1 bis 3 Ethylenoxideinheiten, im Molekül aufweisen. Dazu zählen z. B. Natriumlaurylsulfat, Ammoniumtaurylsulfat, Natriumlaurylethersulfat, Ammoniumlaurylethersulfat, Natriumlaurylsarkosinat, Natriumoleylsuccinat, Ammoniumlaurylsulfosuccinat, Natriumdodecylbenzolsulfonat, Triethanolamindodecylbenzolsulfonat.

Sofern die anionischen Tenside Polyglycoletherketten enthalten, können diese eine konventionelle, vorzugsweise jedoch eine eingeengte Homologenverteilung aufweisen.

Typische Beispiele für kationische Tenside sind quartäre Ammoniumverbindungen, wie beispielsweise das Cetyltrimethylammoniumchlorid, Dimethyldistearylammoniumchlorid, und Esterquats, insbesondere quaternierte Fettsäuretrialkanolaminestersalze.

Geeignete amphotere Tenside sind z. B. Aminopropionate, Aminoglycinate, Imidazoliniumbetaine und Sulfobetaine, Alkylbetaine, Alkylamidopropylbetaine, Alkylsulfobetaine, Alkylglycinate, Alkylcarboxyglycinate, Alkylamphoacetate oder -Propionate, Alkylamphodiacetate oder -dipropionate.

Es ist erfindungsgemäß bevorzugt, wenn der Wirkstoff bzw. die Wirkstoffe Duftstoffe, antimikrobielle Wirkstoffe und/oder Insektenrepellentien sind. Hierbei ist es allerdings nicht ausgeschlossen, dass beispielsweise einige Duftstoffe auch gleichzeitig eine Wirkung gegen Insekten aufweisen, wie dies beispielsweise bei Citronellol und Geraniol der Fall ist. Erfindungsgemäß besonders bevorzugt handelt es sich bei dem Wirkstoff um einen Duftstoff.

Im Rahmen dieses Textes wird unter Duftstoffen eine Substanz oder ein Substanzgemisch verstanden, das olfaktorisch wahrgenommen wird.

Die Auswahl der Duftstoffe ist dabei sehr umfassend; entsprechende Duftstoffe, die erfindungsgemäß eingesetzt werden können, finden sich z. B. in "S. Arctander, Perfume and Flavor Chemicals, Vol. I und II, Montclair, N. J., 1969, Montclair, N. J.Selbstverlag" oder "H. Surburg und J. Panten, Common Fragrance and Flavor Materials, 6th ed., Wiley-VCH, Weinheim, 2016". Im Einzelnen seien im Folgenden Duftstoffe genannt, ausgewählt aus der Gruppe

der Kohlenwasserstoffe, dabei bevorzugt 3-Caren; α-Pinen; β-Pinen; α-Terpinen; γ-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien; Styrol; Diphenylmethan;

der aliphatischen Alkohole, dabei bevorzugt Hexanol; Octanol; 3-Octanol; 2,6-Dimethylheptanol; 2-Methyl-2-heptanol; 2-Methyl-2-octanol; (E)-2-Hexenol; (E)- und (Z)-3-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol; 3,7-DIMETHYL-6-OCTEN-1-OL;

der aliphatischen Aldehyde und deren Acetale, dabei bevorzugt Hexanal; Heptanal; Octanal; Nonanal;

Decanal; Undecanal; Dodecanal; Tridecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal;2,6,10-Trimethyl-9-undecenal; 2,6,10-Trimethyl-5,9-undecadienal; Heptanaldiethylacetal; 1,1-Dimethoxy-2,2,5-trimethyl-4-hexen; Citronellyloxyacetaldehyd; 1-(1-Methoxypropoxy)-(E/Z)-3-hexen;

der aliphatischen Ketone und deren Oxime, dabei bevorzugt 2-Heptanon; 2-Octanon; 3-Octanon; 2-Nonanon; 5-Methyl-3-heptanon; 5-Methyl-3-heptanonoxim; 2,4,4,7-Tetramethyl-6-octen-3-on; 6-Methyl-5-hepten-2-on; 1-(1,2,3,4,5,6,7,8-OCTAHYDRO-2,3,8,8-TETRAMETHYL-2-NAPHTHYL)ETHAN-1-ONE; 3-METHYL-4-(2,6,6-TRIMETHYL-2-CYCLOHEXEN-1-YL)-3-BUTEN-2-ONE

der aliphatischen schwefelhaltigen Verbindungen, dabei bevorzugt 3-Methylthio-hexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;

der aliphatischen Nitrile, dabei bevorzugt 2-Nonensäurenitril; 2-Undecensäurenitril; 2-Tridecensäurenitril; 3,12-Tridecadiensäurenitril; 3,7-Dimethyl-2,6-octadien-säurenitril; 3,7-Dimethyl-6-octensäurenitril;

der Ester von aliphatischen Carbonsäuren, dabei bevorzugt (E)- und (Z)-3-Hexenylformiat; Ethylacetoacetat; Isoamylacetat; Hexylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat; Isoamylbutyrat; Hexylbutyrat; (E)- und (Z)-3-Hexenyl-isobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinat; Methyl-2-noninat; Allyl-2-isoamyloxyacetat; Methyl-3,7-dimethyl-2,6-octadienoat;4-Methyl-2-pentyl-crotonat;

der acyclischen Terpenalkohole, dabei bevorzugt Citronellol; Geraniol; Nerol; Linalool; Lavadulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate und 3-Methyl-2-butenoate;

der acyclischen Terpenaldehyde und -ketone, dabei bevorzugt Geranial; Neral; Citronellal; 7-Hydroxy-3,7-dimethyloctanal; 7-Methoxy-3,7-dimethyloctanal; 2,6,10-Trimethyl-9-undecenal; Geranylaceton; sowie die Dimethyl- und Diethylacetale von Geranial, Neral, 7-Hydroxy-3,7-dimethyloctanal;

der cyclischen Terpenalkohole, dabei bevorzugt Isopulegol; alpha-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate und 3-Methyl-2-butenoate; Menthylformiat; Menthylpropionat; Menthylbutyrat; Menthylisobutyrat; Menthylisovalerianat; Menthylhexanoat; Menthylcrotonat; Menthyltiglinat;

der cyclischen Terpenaldehyde und -ketone, dabei bevorzugt Menthon; Isomenthon; 8-Mercaptomenthan-3-on; Carvon; Campher; Fenchon; alpha-Ionon; beta-Ionon; beta-n-Methylionon; beta-Isomethylionon; alpha-Iron; alpha-Damascon; beta-Damascon; beta-Damascenon; delta-Damascon; gamma-Damascon; 1-(2,4,4-Trimethyl-2-cyclohexen-1-yl)-2-buten-1-on; 1,3,4,6,7,8a-Hexahydro-1,1,5,5-tetramethyl-2H-2,4a-methanonaphthalen-8(5H)-on;2-Methyl-4-(2,6,6-trimethyl-1-cyclohexen-1-yl)-2-butenal; Nootkaton; Dihydro-nootkaton; 4,6,8-Megastigmatrien-3-on; alpha-Sinensal; beta-Sinensal; acetyliertes Ce-dernholzöl (Methylcedrylketon);

der cyclischen Alkohole, dabei bevorzugt 4-tert.-Butylcyclohexanol; 3,3,5-Trimethylcyclohexanol; 3-Isocamphylcyclohexanol; 2,6,9-Trimethyl-Z2,Z5,E9-cyclododecatrien-1-ol; 2-Isobutyl-4-methyltetrahydro-2H-pyran-4-ol;

der cycloaliphatischen Alkohole, dabei bevorzugt alpha,3,3-Trimethylcyclohexylmethanol; 1-(4-Isopropylcyclohexyl)ethanol; 2-Methyl-4-(2,2,3-trimethyl-3-cyclopent-1-yl)butanol; 2-Methyl-4-(2,2,3-trimethyl-3-cyclopent-1 -yl)-2-buten-1 -ol; 2-Ethyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;

der cyclischen und cycloaliphatischen Ether, dabei bevorzugt Cineol; Cedrylmethylether; Cyclododecylmethylether; 1,1-Dimethoxycyclododecan; (Ethoxymethoxy)cyclododecan; alpha-Cedrenepoxid; 3a,6,6,9a-Tetramethyldodecahydronaphtho[2,1-b]furan; 3a-Ethyl-6,6,9a-trimethyldodecahydronaphtho[2,1-b]furan; 1,5,9-Trimethyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;

der cyclischen und makrocyclischen Ketone, dabei bevorzugt 4-tert.-Butylcyclohexanon; 2,2,5-Trimethyl-5-pentylcyclopentanon; 2-Heptylcyclopentanon; 2-Pentylcyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopentadecanon; 4-(1-Ethoxyvinyl)-3,3,5,5-tetramethylcyclohexanon; 4-tert.-Pentylcyclohexanon; 5-Cyclohexadecen-1-on; 6,7-Dihydro-1,1,2,3,3-pentamethyl-4(5H)-indanon; 8-Cyclohexadecen-1-on; 9-Cycloheptadecen-1-on; Cyclopentadecanon; Cyclohexadecanon;

der cycloaliphatischen Aldehyde, dabei bevorzugt 2,4-Dimethyl-3-cyclohexencarbaldehyd; 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;

der cycloaliphatischen Ketone, dabei bevorzugt 1-(3,3-Dimethylcyclohexyl)-4-penten-1-on; 2,2-Dimethyl-1-(2,4-dimethyl-3-cyclohexen-1-yl)-1-propanon; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert.-Butyl-(2,4-dimethyl-3-cyclohexen-1-yl)keton;

der Ester cyclischer Alkohole, dabei bevorzugt 2-tert-Butylcyclohexylacetat; 4-tert-Butylcyclohexylacetat; 2-tert-Pentylcyclohexylacetat; 4-tert-Pentylcyclohexylacetat; 3,3,5-Trimethylcyclohexylacetat; Decahydro-2-naphthylacetat; 2-Cyclopentylcyclopentylcrotonat; 3-Pentyltetrahydro-2H-pyran-4-ylacetat; Decahydro-2,5,5,8a-tetramethyl-2-naphthylacetat; 4,7-Methano-3a,4,5,6,7,7a-hexahydro-5, bzw. 6-indenylacetat; 4,7-Methano-3a,4,5,6,7,7a-hexahydro-5, bzw. 6-indenylpropionat; 4,7-Methano-3a,4,5,6,7,7a-hexahydro-5, bzw. 6-indenylisobutyrat; 4,7-Methanooctahydro-5, bzw. 6-indenylacetat;

der Ester cycloaliphatischer Alkohole, vorzugsweise 1-Cyclohexylethylcrotonat;

der Ester cycloaliphatischer Carbonsäuren, dabei bevorzugt Allyl-3-cyclohexylpropionat; Allylcyclohexyloxyacetat; cis- und trans-Methyldihydrojasmonat; cis- und trans-Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat;

der araliphatischen Alkohole, dabei bevorzugt Benzylalkohol; 1-Phenylethylalkohol; 2-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenylpropanol; 2-Methyl-5-phenylpentan-1-ol; 3-Methyl-5-phenylpentanol; 3-Phenyl-2-propen-1-ol; 4-Methoxybenzylalkohol; 1-(4-Isopropylphenyl)ethanol;

der Ester von araliphatischen Alkoholen und aliphatischen Carbonsäuren, dabei bevorzugt Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat;

der araliphatischen Ether, dabei bevorzugt 2-Phenylethylmethylether; 2-Phenylethylisoamylether; 2-Phenylethyl-1-ethoxyethylether; Phenylacetaldehyddimethylacetal; Phenylacetaldehyddiethylacetal; Hydratropaaldehyddimethylacetal; Phenylacetaldehydglycerinacetal; 2,4,6-Trimethyl-4-phenyl-1,3-dioxan; 4,4a,5,9b-Tetrahydroindeno[1,2-d]-m-dioxin; 4,4a,5,9b-Tetrahydro-2,4-dimethylindeno[1,2-d]-m-dioxin;

der aromatischen und araliphatischen Aldehyde, dabei bevorzugt Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-tert.-butylphenyl)propanal; 2-Methyl-3-(4-isobutylphenyl)propanal; 3-(4-tert.-Butylphenyl)propanal; Zimtaldehyd; alpha-Butylzimtaldehyd; alpha-Amylzimtaldehyd; alpha-Hexylzimtaldehyd; 3-Methyl-5-phenylpentanal; 4-Methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;

der aromatischen und araliphatischen Ketone, dabei bevorzugt Acetophenon; 4-Methylacetophenon; 4-Methoxyacetophenon; 4-tert.-Butyl-2,6-dimethylacetophenon; 4-Phenyl-2-butanon; 4-(4-Hydroxyphenyl)-2-butanon; 1-(2-Naphthalenyl)ethanon;2-Benzofuranylethanon;(3-Methyl-2-benzofuranyl)ethanon; Benzophenon; 1,1,2,3,3,6-Hexamethyl-5-indanylmethylketon; 6-tert.-Butyl-1,1-dimethyl-4-indanylmethylketon; 1-[2,3-dihydro-1,1,2,6-tetramethyl-3-(1-methylethyl)-1H-5-indenyl]ethanon; 5',6',7',8'-Tetrahydro-3',5',5',6',8',8'-hexamethyl-2-acetonaphthon;

der aromatischen und araliphatischen Carbonsäuren und deren Ester, dabei bevorzugt Benzoesäure; Phenylessigsäure; Methylbenzoat; Ethylbenzoat; Hexylbenzoat; Methylphenylacetat; Ethylphenylacetat; Geranylphenylacetat; Phenylethyl-phenylacetat; Methylcinnmat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;

der stickstoffhaltigen aromatischen Verbindungen, dabei bevorzugt 2,4,6-Trinitro-1,3-dimethyl-5-tert.-butylbenzol; 3,5-Dinitro-2,6-dimethyl-4-tert.-butylacetophenon; Zimtsäurenitril; 3-Methyl-5-phenyl-2-pentensäurenitril; 3-Methyl-5-phenylpentansäurenitril; Methylanthranilat; Methy-N-methylanthranilat; Schiff'sche Basen von Methylanthranilat mit 7-Hydroxy-3,7-dimethyloctanal, 2-Methyl-3-(4-tert.-butylphenyl)propanal oder 2,4-Dimethyl-3-cyclohexencarbaldehyd; 6-Isopropylchinolin; 6-Isobutylchinolin; 6-sec.-Butylchinolin; 2-(3-Phenylpropyl)pyridin; Indol; Skatol; 2-Methoxy-3-isopropylpyrazin; 2-Isobutyl-3-methoxypyrazin;

der Phenole, Phenylether und Phenylester, dabei bevorzugt Estragol; Anethol; Eugenol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat;

der heterocyclischen Verbindungen, dabei bevorzugt 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;

der Lactone, dabei bevorzugt 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid; 4-Methyl-1,4-decanolid; 1,15-Pentadecanolid; cis- und trans-11-Pentadecen-1,15-olid; cis- und trans-12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,13-tridecandioat; Cumarin; 2,3-Dihydrocumarin; Octahydrocumarin.

Bei den erfindungsgemäß verwendeten Duftstoffen kann es sich ebenfalls um ätherische Öle, Concretes, Absolues, Resine, Resinoide, Balsame und/oder Tinkturen handeln.

Bevorzugte Etherische Öle, Concretes, Absolues, Resine, Resinoide, Balsame und/oder Tinkturen sind bevorzugt auszuwählen aus der Gruppe bestehend aus:

Ambratinktur; Amyrisöl; Angelicasamenöl; Angelicawurzelöl; Anisöl; Baldrianöl; Basilikumöl; Baummoos-Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-absolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptus-citriodora-Öl; Eucalyptusöl; Fenchelöl; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl; Lavendel-Absolue; Lavendelöl; Lemongrasöl; Liebstocköl; Limetteöl destilliert; Limetteöl gepreßt; Linaloeöl; Litseacubeba-Öl; Lorbeerblätteröl; Macisöl; Majoranöl; Mandarinenöl; Massoirindenöl; Mimosa-Absolue; Moschuskörneröl; Moschustinktur; Muskateller-Salbei-Öl; Muskatnußöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl (engl.: pine oil); Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetes-öl; Tannennadelöl; Teebaum-Öl; Terpentinöl (engl.: turpentine oil); Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl.

Eine erfindungsgemäße Verwendung ist bevorzugt, wobei die einen Wirkstoff enthaltene flüssigen Zusammensetzung zusätzlich ein oder mehrere Konservierungsmittel, vorzugsweise Natriumbenzoat umfasst.

Eine erfindungsgemäße Verwendung ist bevorzugt, wobei die einen Wirkstoff enthaltene flüssigen Zusammensetzung

5 bis 90 Gew.-%, vorzugsweise 6 bis 70 Gew.-%, besonders bevorzugt 8 bis 65 Gew.-% eines Lösungsmittels umfasst,

1 bis 25 Gew.-% vorzugsweise 2 bis 20 Gew.-%, besonders bevorzugt 4 bis 10 Gew.-% eines oder mehrerer Wirkstoffe, und

0 bis 5 Gew.-% weitere Additive, insbesondere Konservierungsmittel,

umfasst oder daraus besteht, jeweils bezogen auf das Gesamtgewicht der flüssigen Zusammensetzung.

Eine erfindungsgemäße Verwendung ist bevorzugt, wobei die einen Wirkstoff enthaltene flüssigen Zusammensetzung

5 bis 90 Gew.-%, vorzugsweise 6 bis 70 Gew.-%, besonders bevorzugt 8 bis 65 Gew.-% eines Lösungsmittels umfasst, bei dem es sich nicht um Wasser handelt und das vorzugsweise mit Wasser mischbar ist,

1 bis 25 Gew.-% vorzugsweise 2 bis 20 Gew.-%, besonders bevorzugt 4 bis 10 Gew.-% eines oder mehrerer Wirkstoffe,

10 bis 90 Gew.-%, vorzugsweise 20 bis 85 Gew.-%, besonders bevorzugt 25 bis 85 Gew.-% Wasser,

0 bis 5 Gew.-% weitere Additive, insbesondere Konservierungsmittel, und

0 bis 20 Gew.-% vorzugsweise 0,5 bis 15 Gew.-%, besonders bevorzugt 1,0 bis 10 Gew.-% Tensid

umfasst oder daraus besteht, jeweils bezogen auf das Gesamtgewicht der flüssigen Zusammensetzung.

Unter einem mit Wasser mischbaren Lösungsmittel wird im Rahmen der vorliegenden Erfindung ein Lösungsmittel verstanden, das bei einer Temperatur von 25 °C in jedem Verhältnis mit Wasser gemischt werden kann und hierbei vollständig eine homogene Phase ausbildet.

Eigene Untersuchungen haben gezeigt, dass es bevorzugt ist, wenn das Verhältnis zwischen sämtlichen Wirkstoffen und dem Tensid im Bereich zwischen 1 : 2 und 2 : 1 ist.

Eine erfindungsgemäße Verwendung ist bevorzugt, wobei die einen Wirkstoff enthaltene flüssigen Zusammensetzung

10 bis 90 Gew.-% Ethanol und/oder Dipropylenglycolmethylether

1 bis 25 Gew.-% eines oder mehrerer Wirkstoffe,

10 bis 90 Gew.-% Wasser und
und

0 bis 10 Gew.-% Tensid, das einen nach Griffin berechneten HLB-Wert im Bereich von 3 bis 20 aufweist,

0 bis 5 Gew.-% weitere Additive, insbesondere Konservierungsmittel,

umfasst oder daraus besteht, jeweils bezogen auf das Gesamtgewicht der flüssigen Zusammensetzung.

Es ist erfindungsgemäß bevorzugt, wenn die Zusammensetzung eine homogene Phase ausbildet.

In einer Ausgestaltung der vorliegenden Erfindung besteht die flüssige Zusammensetzung aus zwei oder drei Phasen. Dies kann beispielsweise erzielt werden, indem nicht ineinander lösbare Lösungsmittel eingesetzt werden. Die unterschiedlichen Phasen können unterschiedlich eingefärbt werden, um die die Optik der Zusammensetzung zu verbessern. Es ist erfindungsgemäß bevorzugt, wenn jede der Phasen homogen ausgebildet ist, d.h. die Phasen bestehen nicht aus einer Emulsion. In einer bevorzugten Ausgestaltung der vorliegenden Erfindung besteht die flüssige Zusammensetzung aus einer einzigen Phase.

Ein weiterer Aspekt der vorliegenden Erfindung betrifft eine Vorrichtung (2) zum Freisetzen eines Wirkstoffes in die Umgebung, bestehend aus oder umfassend

    a) eine flüssige Zusammensetzung (3) bestehend aus oder umfassend

        a1) ein oder mehrere Lösungsmittel,

        a2) ein oder mehrere Wirkstoffe,

        a3) optional ein oder mehrere Tenside und

        a4) optional ein oder mehrere Konservierungsmittel

    b) mindestens ein Diffusionsstäbchen (1, 1', 1") wie oben im Text definiert
    und

    c) einen Behälter (9), der zumindest eine Behälteröffnung (10) aufweist und die flüssige Zusammensetzung (3) umfasst.

Über die Länge, den Durchmesser und die Anzahl der Diffusionsstäbchen kann die Diffusions- und Verdunstungsrate der Zusammensetzung aus der Vorrichtung gesteuert werden. Üblicherweise werden drei, vier, fünf, sechs, sieben, acht oder neun Diffusionsstäbchen eingesetzt. Zudem wird die Anzahl, die Länge und der Durchmesser auch an die Menge bzw. Behältergröße angepasst.

Es ist erfindungsgemäß bevorzugt, wenn der Behälter (9) ein Glasbehälter, Keramikbehälter oder Metallbehälter ist. Diese Behälter haben den Vorteil, dass sie ausgewaschen und wiederverwendet werden können und dabei keine Gerüche aufnehmen. Zudem besteht bei diesen Materialine nicht die Gefahr, dass es zu einer Verfärbung der Flasche kommt. Alternativ zu Glas besteht auch die - weniger bevorzugte - Möglichkeit, dass ein Behälter aus einem Kunststoff, vorzugsweise einem transparenten Kunststoff verwendet wird. Erfindungsgemäß können somit Behälter aus Glas, Keramik, Metall oder Kunststoff verwendet werden. Es ist erfindungsgemäß bevorzugt, wenn der Behälter ein Füllvolumen (Innenvolumen) im Bereich von 25 bis 250 mL aufweist, vorzugsweise im Bereich von 40 bis 220 mL. Üblicherweise werden Behälter mit einem Füllvolumen von 50 mL $\pm$ 15 mL, 90 mL $\pm$ 15 mL, 100 mL $\pm$ 15 mL, 120 mL $\pm$ 15 mL, 200 mL $\pm$ 15 mL oder 250 mL $\pm$ 15 mL eingesetzt.

Es ist erfindungsgemäß bevorzugt, wenn das Volumen der flüssigen Zusammensetzung im Behälter im Bereich von 25 bis 500 mL liegt, vorzugsweise im Bereich von 40 bis 300 mL, weiter bevorzugt im Bereich 50 bis 270 mL.

Eine Vorrichtung ist erfindungsgemäß bevorzugt, wobei der Behälter die flüssige Zusammensetzung (3) umfasst und das oder die Diffusionsstäbchen dazu vorgesehen sind, teilweise in die flüssige Zusammensetzung einzutauchen und sich durch die Behälteröffnung aus dem Behälter zu erstrecken. Üblicherweise werden erfindungsgemäße Vorrichtungen so verkauft, dass das oder die Diffusionsstäbchen separat von dem mit der flüssigen Zusammensetzung gefüllten Behälter verpackt sind. So werden die Diffusionsstäbchen meist lose oder zusammengebunden in einem Karton mit dem Behälter verpackt angeboten. Der Konsument öffnet zum Gebrauch den Verschluss des Behälters, entsorgt diesen und steckt die Diffusionsstäbchen durch die Behälteröffnung in die flüssige Zusammensetzung, sodass sich die in die flüssige Zusammensetzung eingetauchte Diffusionsstäbchen durch die Behälteröffnung aus dem Behälter erstrecken. Es ist somit erfindungsgemäß bevorzugt, wenn die Diffusionsstäbchen vor der Nutzung der Vorrichtung nicht bereits mit der flüssigen Zusammensetzung beladen sind und diese erst aufnehmen, wenn der Konsument die Vorrichtung nutzt.

Eine Vorrichtung ist erfindungsgemäß bevorzugt, wobei der Behälter die flüssige Zusammensetzung (3) umfasst und das oder die Diffusionsstäbchen teilweise in die flüssige Zusammensetzung eintauchen und sich durch die Behälteröffnung aus dem Behälter erstrecken.

Eine Vorrichtung ist erfindungsgemäß bevorzugt, bei der das Diffusionsstäbchen bzw. die Diffusionsstäbchen an einem aufnehmenden Endbereich des Diffusionsstäbchens in die flüssige Zusammensetzung (3) eintaucht, um die flüssige Zusammensetzung aufzunehmen, und die flüssige Zusammensetzung an einem abgebenden Endbereich des Diffusionsstäbchens teilweise oder vollständig in die Umgebung abgegeben wird. Die Diffusionsstäbchen nehmen die flüssige Zusammensetzung an dem aufnehmenden Endbereich auf und befördern diese entlang des Diffusionsstäbchens bis zum abgebenden Endbereich, wo sie die flüssige Zusammensetzung vollständig oder teilweise in die Umgebung abgeben. Üblicherweise und erfindungsgemäß bevorzugt befindet sich die flüssige Zusammensetzung dabei in einem Behälter (9), der zumindest eine Behälteröffnung (10) aufweist, und die Diffusionsstäbchen erstrecken sich durch die Behälteröffnung (10) aus dem Behälter (9).

Es ist erfindungsgemäß besonders bevorzugt, wenn die Diffusionsstäbchen lotrecht (als auch senkrecht oder vertikal bezeichnet) oder überwiegend (bevorzugt max. 45° Abweichung zum Lot, vorzugsweise max. zur 30° Abweichung zum Lot, weiter bevorzugt max. 25° Abweichung zum Lot) lotrecht angeordnet sind. Die flüssige Zusammensetzung wird dabei entgegen der Schwerkraft vom aufnehmenden Endbereich des Diffusionsstäbchens bis zum abgebenden Endbereich des Diffusionsstäbchens transportiert.

Der Transport der flüssigen Zusammensetzung erfolgt dabei überwiegend entlang der Diffusionsstäbchen, wobei die flüssige Zusammensetzung an dem Endbereich die flüssige Zusammensetzung aufnimmt an dem Sie in die flüssige Zusammensetzung eintaucht und die flüssige Zusammensetzung an dem anderen Endbereich des Diffusionsstäbchens in die Umgebung abgibt.

Ein weiterer Aspekt der vorliegenden Erfindung betrifft ein Kit zur Herstellung einer Vorrichtung (2) zum Freisetzen eines Wirkstoffes in die Umgebung, bestehend aus oder umfassend

a1) ein oder mehrere Lösungsmittel,

a2) ein oder mehrere Wirkstoffe,

a3) optional ein oder mehrere Tenside und

a4) optional ein oder mehrere Konservierungsmittel

b) mindestens ein Diffusionsstäbchen (1, 1', 1") wie oben im Text definiert
und

c) optional einen Behälter (9), der zumindest eine Behälteröffnung (10) aufweist und die flüssige Zusammensetzung (3) umfasst.

Ein erfindungsgemäßes Kit umfasst vorzugsweise drei, vier, fünf, sechs, sieben, acht oder neun Diffusionsstäbchen.

Durch Hinzufügen von Wasser und ggf. Durchmischen der Komponenten kann aus einem erfindungsgemäßen Kit eine erfindungsgemäße Vorrichtung (2) erhalten werden.

Ein erfindungsgemäßes Kit ist besonders bevorzugt, das zusätzlich c) einen Behälter (2) umfasst, der zumindest eine Behälteröffnung (10) aufweist. Hierbei ist es erfindungsgemäß bevorzugt, wenn die Zusammensetzung des Kits bereits in dem Behälter (10) enthalten ist. Der Verbraucher kann dann Wasser in den Behälter geben, um eine gebrauchsfertige Zusammensetzung zu erhalten, wobei vorzugsweise eine entsprechende Markierung in dem Behälter vorhanden ist, bis zur der das Wasser aufgefüllt werden muss. Alternativ besteht natürlich die Möglichkeit, dass dem Kit kein entsprechender Behälter zugegeben wird und der Verbraucher ein eigenes Behältnis verwendet oder einen in einem vorherigen Kit enthaltenen Behälter (wieder)verwendet. Sofern das Kit keinen entsprechenden Behälter umfasst, der eine Behälteröffnung (10) aufweist und ein Ausreichendes Innenvolumen aufweist, das die Zusammensetzung nebst Wasser aufnehmen kann, wird die Zusammensetzung vorzugsweis in einem kleineren Behälter, aus Keramik, Metall, Glas oder Kunststoffbehälter, oder einem Beutel abgefüllt.

Ein weiterer Aspekt der vorliegenden Erfindung betrifft eine ein Diffusionsstäbchen wie weiter oben im Zusammenhang mit der Verwendung des Diffusionsstäbchens beschrieben.

Die Erfindung ist nachstehend anhand von Zeichnung beispielhaft erläutert. Diese zeigt in

Fig.1      ein erfindungsgemäß verwendetes Diffusionsstäbchen, das durch Aufrollen von Papier hergestellt wurde und eine Seele (5) umfasst,

Fig. 2     ein Diffusionsstäbchen (1), bei dem das Papier (4) teilweise abgerollt ist.

Fig. 3     ein Papier zur Herstellung eines erfindungsgemäßen Diffusionsstäbchens, im Querschnitt, wobei das Papier einseitig gestrichen ist,

Fig. 4     ein Papier zur Herstellung eines erfindungsgemäßen Diffusionsstäbchens, im Querschnitt, wobei das Papier beidseitig gestrichen ist,

Fig. 5     ein erfindungsgemäß verwendetes Diffusionsstäbchen, das durch Aufrollen von Papier hergestellt wurde und eine Seele (4) umfasst, bei dem die Durchmesser (d, D) die Wanddicke (W) die Länge (L) und die Achse (A) eingezeichnet sind

und

Fig. 6     eine erfindungsgemäße Vorrichtung (2) zum Freisetzen eines Wirkstoffes.

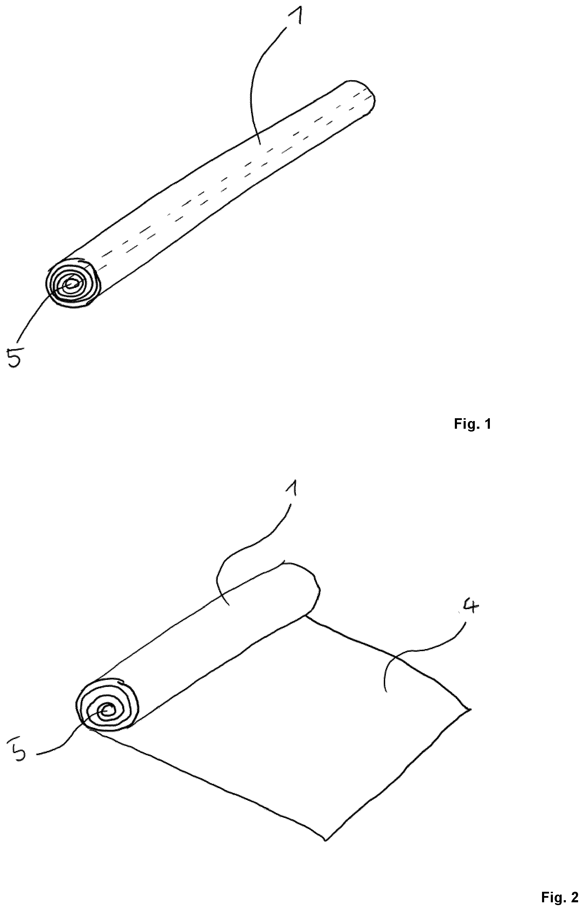

Figur 1 zeigt ein Diffusionsstäbchen (1), das durch Aufrollen von Papier hergestellt wurde. Bei dem Papier kann es sich beispielsweise um ein ungebleichtes Zuckerrohrpapier handeln. Das Diffusionsstäbchen (1) weist eine hohle Seele (5) auf, die sich parallel zur Längsachse (nicht eingezeichnet) erstreckt. Der Durchmesser der Seele kann beispielsweise 1,8 mm betragen, während der Durchmesser des Diffusionsstäbchens (1) 3,8 mm beträgt. Es ist bevorzugt, wenn das Material des Diffusionsstäbchens eine Dichte im Bereich von 0,7 bis 0,85 g/cm$^3$ aufweist.

Fig. 2 zeigt ein Diffusionsstäbchen (1), bei dem das Papier (4) teilweise abgerollt ist und das Diffusionsstäbchen eine hohle Seele (5) aufweist.

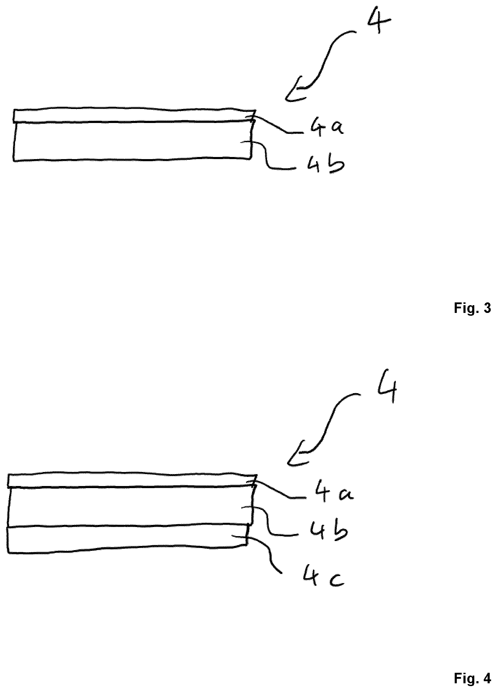

Fig. 3 zeigt schematisch den Aufbau eines Papiers (4), das einseitig gestrichen (beschichtet) ist. Hierbei wird das Rohpapier (4b) mit einem Strich beschichtet und unter Ausbildung einer Schicht (4a) getrocknet. Der Strich kann beispielsweise Stärke als Bindemittel und Kaolin als anorganisches Bindemittel enthalten.

Fig. 4 zeigt schematisch den Aufbau eines Papiers (4), das beidseitig gestrichen (beschichtet) ist. Hierbei wird das Rohpapier (4b) mit einem ersten Strich beschichtet und unter Ausbildung einer Schicht (4a) getrocknet. Der erste Strich kann beispielsweise Stärke als Bindemittel und Kaolin als anorganisches Bindemittel enthalten. Anschließend wird das einseitig beschichtete Papier auf der anderen Seite mit einem zweiten Strich beschichten und unter Ausbildung einer Schicht (4b) getrocknet. Der zweite Strich kann beispielsweise Stärke als Bindemittel und Kaolin als anorganisches Bindemittel enthalten. Der erste Strich und zweite Strich können identisch oder unterschiedlich ausgebildet sein.

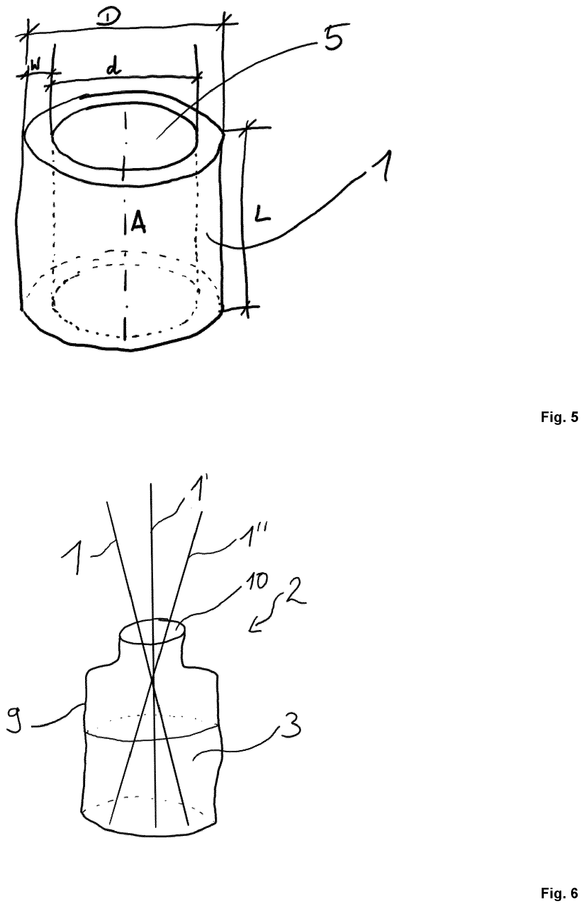

Fig. 5 zeigt schematisch den Aufbau eines erfindungsgemäß verwendeten Diffusionsstäbchens, das durch Aufrollen von Papier hergestellt wurde und eine Seele (5) umfasst, bei dem die Durchmesser (d, D) die Wanddicke (W) die Länge (L) und die Achse (A) eingezeichnet sind.

Fig. 6 zeigt eine erfindungsgemäße Vorrichtung (2) zum Freisetzen einer einen Wirkstoff enthaltenen flüssigen Zusammensetzung in die Umgebung in einer schematischen Darstellung. In einem Behälter (9), der eine Behälteröffnung (10) aufweist, befindet sich eine flüssige Zusammensetzung (3). Die flüssige Zusammensetzung (3) umfasst beispielsweise Wasser, ein mit Wasser mischbares Lösungsmittel (Ethanol oder Dipropylenglycolmethylether) und ein oder mehrere Wirkstoffe (hier Duftstoffe). In dem Behälter sind drei Diffusionsstäbchen (1, 1', 1") angeordnet, die in Kontakt mit der in dem Behälter befindlichen flüssigen Zusammensetzung (3) stehen und sich durch die Behälteröffnung (10) aus dem Behälter (9) erstrecken. Es können allerdings auch mehr oder weniger Diffusionsstäbchen verwendet werden. Bei den Diffusionsstäbchen (1, 1', 1") handelt es sich um Diffusionsstäbchen, die durch Aufrollen von Papier hergestellt wurden und eine Seele aufweisen (hier nicht abgebildet). Alternativ können auch Diffusionsstäbchen eingesetzt werden, die durch Aufrollen von Papier hergestellt wurden und die keine Seele aufweisen.

**Beispiel 1:**

Es wird eine Zusammensetzung erstellt, die 60 Gew.-% Dipropylenglycolmethylether, 30 Gew.-% Wasser und 10 Gew.-% Duftstoff umfasst. Diese Zusammensetzung wird in eine zylindrische Glasflasche überführt. Durch die Öffnung der Glasflasche werden fünf erfindungsgemäße Diffusionsstäbchen, die durch Aufrollen von Papier (4) hergestellt wurden, eingeführt, sodass die flüssige Zusammensetzung über die Diffusionsstäbchen aus der Glasflasche transportiert und an der Oberfläche der Diffusionsstäbchen verdampfen kann. Die eingesetzten Diffusionsstäbchen haben einen Durchmesser (D) von 3 mm, eine Länge (L) von 240 mm, keine Seele und eine Dichte von ca. 0,95 g/cm$^3$.

Die Duftintensität ist über die gesamte Laufzeit sehr gut.

**Beispiel 2:**

Es wird eine Zusammensetzung erstellt, die 60 Gew.-% Dipropylenglycolmethylether, 30 Gew.-% Wasser und 10 Gew.-% Duftstoff umfasst. Diese Zusammensetzung wird in eine zylindrische Glasflasche überführt. Durch die Öffnung der Glasflasche werden fünf erfindungsgemäße Diffusionsstäbchen, die durch Aufrollen von Papier (4) hergestellt wurden, eingeführt, sodass die flüssige Zusammensetzung über die Diffusionsstäbchen aus der Glasflasche transportiert und an der Oberfläche der Diffusionsstäbchen verdampfen kann. Die eingesetzten Diffusionsstäbchen haben einen Durchmesser (D) von 3,8 mm, eine Länge (L) von 240 mm, eine Seele mit einem Durchmesser (d) von 0,8 und eine Dichte von ca. 0,95 g/cm$^3$.

Die Duftintensität ist über die gesamte Laufzeit sehr gut. Im Vergleich mit Beispiel 2 wurde die Zusammensetzung schneller verdampft.

**Beispiel 3:**

Es wird eine Zusammensetzung erstellt, die 60 Gew.-% Dipropylenglycolmethylether, 30 Gew.-% Wasser und 10 Gew.-% Duftstoff umfasst. Diese Zusammensetzung wird in eine zylindrische Glasflasche überführt. Durch die Öffnung der Glasflasche werden fünf erfindungsgemäße Diffusionsstäbchen, die durch Aufrollen von Papier (4) hergestellt wurden, eingeführt, sodass die flüssige Zusammensetzung über die Diffusionsstäbchen aus der Glasflasche transportiert und an der Oberfläche der Diffusionsstäbchen verdampfen kann. Die eingesetzten Diffusionsstäbchen haben einen Durchmesser (D) von 3 mm, eine Länge (L) von 240 mm, keine Seele und eine dichte von 0,79 g/cm$^3$.

Die Duftintensität ist über die gesamte Laufzeit sehr gut. Im Vergleich mit Beispiel 1 wurde die Zusammensetzung schneller verdampft.

Beispiel 4:

Es wird eine Zusammensetzung erstellt, die 60 Gew.-% Dipropylenglycolmethylether, 30 Gew.-% Wasser und 10 Gew.-% Duftstoff umfasst. Diese Zusammensetzung wird in eine zylindrische Glasflasche überführt. Durch die Öffnung der Glasflasche werden fünf erfindungsgemäße Diffusionsstäbchen, die durch Aufrollen von Papier (4) hergestellt wurden, eingeführt, sodass die flüssige Zusammensetzung über die Diffusionsstäbchen aus der Glasflasche transportiert und an der Oberfläche der Diffusionsstäbchen verdampfen kann. Die eingesetzten Diffusionsstäbchen haben einen Durchmesser (D) von 3,8 mm, eine Länge (L) von 240 mm, eine Seele mit einem Durchmesser (d) von 0,8 und eine Dichte von 0,79 g/cm$^3$.

Die Duftintensität ist über die gesamte Laufzeit sehr gut. Im Vergleich mit Beispiel 1, 2 und 3 wurde die Zusammensetzung schneller verdampft.

Beispiel 5:

Es wird eine Zusammensetzung erstellt, die 10 Gew.-% Ethanol, 80 Gew.-% Wasser, 5 Gew.-% Tensid und 5 Gew.-% Duftstoff umfasst. Diese Zusammensetzung wird in eine zylindrische Glasflasche überführt. Durch die Öffnung der Glasflasche werden vier erfindungsgemäße Diffusionsstäbchen, die durch Aufrollen von Papier (4) hergestellt wurden, eingeführt, sodass die flüssige Zusammensetzung über die Diffusionsstäbchen aus der Glasflasche transportiert und an der Oberfläche der Diffusionsstäbchen verdampfen kann.

Die Duftintensität ist über die gesamte Laufzeit sehr gut.

Bezugszeichenliste:

| | |
|---|---|
| 1, 1', 1" | Diffusionsstäbchen |
| 2 | Vorrichtungen zum Freisetzen flüssigen Zusammensetzung |
| 3 | flüssigen Zusammensetzung |
| 4 | Papier |
| 4b | Trägerpapier |
| 4a,4c | Beschichtung |
| 5 | Seele |
| 6 | Wand |
| 9 | Behälter |
| 10 | Behälteröffnung |
| A | Achse des Diffusionsstäbchens (1) |
| d | Durchmesser der Seele (5) bzw. Innendurchmesser des Diffusionsstäbchens |
| D | Durchmesser des Stäbchens |
| L | Länge des Diffusionsstäbchens (1) |
| W | Wanddicke |

**Patentansprüche**

**1.** Verwendung eines Diffusionsstäbchens (1) in Vorrichtungen (2) zum Freisetzen einer einen Wirkstoff enthaltenen flüssigen Zusammensetzung (3), wobei das Diffusionsstäbchen (1) durch Aufrollen von Papier (4) hergestellt wurde.

**2.** Verwendung nach Anspruch 1, wobei das Diffusionsstäbchen teilweise in die flüssige Zusammensetzung eintaucht.

**3.** Verwendung nach Anspruch 1 oder 2, wobei das Diffusionsstäbchen an einem aufnehmenden Endbereich des Diffusionsstäbchens in die flüssige Zusammensetzung (3) eintaucht, um die flüssige Zusammensetzung aufzunehmen, und die flüssige Zusammensetzung an einem abgebenden Endbereich des Diffusionsstäbchens teilweise oder vollständig in die Umgebung abgegeben wird.

**4.** Verwendung nach einem der vorherigen Ansprüche, wobei das Diffusionsstäbchen (1) eine hohle Seele (5) entlang seiner Achse aufweist.

**5.** Verwendung nach Anspruch 4, wobei die Seele einen Durchmesser (d) im Bereich von 0,50 mm bis 3,0 mm aufweist, vorzugsweise im Bereich von 0,60 mm bis 3,0 mm aufweist, weiter bevorzugt im Bereich von 0,70 mm bis 2,0 mm aufweist.

**6.** Verwendung nach einem der vorherigen Ansprüche, wobei das Diffusionsstäbchen (1) einen Durchmesser (D) im Bereich von 1,0 bis 10,0 mm aufweisen, vorzugsweise im Bereich von 2,5 bis 7,0 mm aufweisen, besonders bevorzugt im Bereich von 3,0 bis 5,0 mm aufweisen.

**7.** Verwendung nach einem der vorherigen Ansprüche, wobei das Diffusionsstäbchen eine Länge (L) im Bereich von 50 bis 300 mm aufweisen, vorzugsweise im Bereich von 80 bis 260 mm aufweisen, besonders bevorzugt im Bereich von 90 bis 240 mm aufweisen, weiter bevorzugt im Bereich von 170 bis 240 mm aufweisen.

**8.** Verwendung nach einem der der Ansprüche 4 bis 7, wobei das Diffusionsstäbchen (1) eine Wanddicke (W) im Bereich von 0,4 bis 3,4 mm aufweist, vorzugsweise im Bereich von 0,5 bis 2,0, besonders bevorzugt im Bereich von 0,7 bis 2,5 mm.

**8.** Verwendung nach einem der vorherigen Ansprüche, wobei die Wand des Diffusionsstäbchens (1) aus 3 bis 55 Papierschichten besteht, vorzugsweise aus 5 bis 25 Papierschichten, weiter bevorzugt aus 6 bis 16 Papierschichten

**9.** Verwendung nach einem der vorherigen Ansprüche, wobei das Papier (4) gebleicht ist.

**10.** Verwendung einem der vorherigen Ansprüche, wobei die Zusammensetzung (3) Wasser umfasst.

**11.** Verwendung einem der vorherigen Ansprüche, wobei die Zusammensetzung (3) mindestens ein Lösungsmittel umfasst, ausgewählt aus der Gruppe bestehend aus Dipropylenglycolmethylether, Dipropylenglycolmethylether-acetat Diethylenglycolmonoethylether und Ethanol umfasst.

**12.** Verwendung nach einem der vorherigen Ansprüche, wobei die Zusammensetzung (3) zusätzlich ein Tensid enthält.

**13.** Vorrichtung (2) zum Freisetzen eines Wirkstoffes in die Umgebung, bestehend aus oder umfassend

    a) eine flüssige Zusammensetzung (3) bestehend aus oder umfassend

        a1) ein oder mehrere Lösungsmittel,
        a2) ein oder mehrere Wirkstoffe,
        a3) optional ein oder mehrere Tenside und
        a4) optional ein oder mehrere Konservierungsmittel

    b) mindestens ein Diffusionsstäbchen (1, 1', 1") nach einem der Ansprüche 1 bis 21
    und
    c) einen Behälter (9), der zumindest eine Behälteröffnung (10) aufweist und die flüssige Zusammensetzung (3) umfasst.

**14.** Vorrichtung nach Anspruch 13, wobei

    a) das oder die Diffusionsstäbchen dazu vorgesehen sind, teilweise in die flüssige Zusammensetzung einzutauchen und sich durch die Behälteröffnung aus dem Behälter zu erstrecken
    und/oder
    b) das oder die Diffusionsstäbchen teilweise in die flüssige Zusammensetzung eintauchen und sich durch die Behälteröffnung aus dem Behälter erstrecken.

**15.** Vorrichtung nach Anspruch 13 oder 14, wobei das Volumen der flüssigen Zusammensetzung im Behälter im Bereich von 25 bis 500 mL liegt, vorzugsweise im Bereich von 40 bis 300 mL, weiter bevorzugt im Bereich 50 bis 270 mL

Fig. 1

Fig. 2

4

4a

4b

Fig. 3

4

4a

4b

4c

Fig. 4

**Fig. 5**

**Fig. 6**

Europäisches Patentamt
European Patent Office
Office européen des brevets

# EUROPÄISCHER RECHERCHENBERICHT

**Nummer der Anmeldung**

EP 23 15 4975

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2011/262377 A1 (MCKAY NICHOLAS [US] ET AL) 27. Oktober 2011 (2011-10-27) | 1,4-14 | INV. B31D5/00 A61L9/00 A61L9/12 B32B1/00 B32B3/26 D21H21/14 |
| A | * Absätze [0015], [0028], [0048], [0049], [0069], [0078], [0080], [0084], [0087], [0092], [0095], [0096], [0126], [0138], [0163] * * Absätze [0140], [0145], [0158] * ----- | 2,3,15 | |
| X | US 2021/205489 A1 (HSIAO MING JEN [TW]) 8. Juli 2021 (2021-07-08) | 1,4,14 | |
| A | * Absätze [0013], [0018], [0045], [0047], [0048], [0049], [0055], [0058]; Abbildung 2 * ----- | 5-13 | |
| X | CN 113 041 383 A (DONGGUAN YIH TEH ELECTRIC PRODUCTS CO LTD) 29. Juni 2021 (2021-06-29) | 1,2,4,9, 14 | |
| A | * Absätze [0019], [0046], [0062], [0063]; Anspruch 5; Abbildungen 1,2,3,8 * ----- | 3,5-8, 10-13,15 | |

**RECHERCHIERTE SACHGEBIETE (IPC)**

B31D
B31F
B32B
A61L
D21H
B31C

Der vorliegende Recherchenbericht wurde für alle Patentansprüche erstellt

| Recherchenort | Abschlußdatum der Recherche | Prüfer |
|---|---|---|
| Den Haag | 21. September 2023 | Van Beurden-Hopkins |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 23 15 4975

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

21-09-2023

| Im Recherchenbericht angeführtes Patentdokument | Datum der Veröffentlichung | Mitglied(er) der Patentfamilie | Datum der Veröffentlichung |
|---|---|---|---|
| US 2011262377 A1 | 27-10-2011 | CN 103037907 A | 10-04-2013 |
| | | EP 2552499 A1 | 06-02-2013 |
| | | JP 2013526906 A | 27-06-2013 |
| | | KR 20130100049 A | 09-09-2013 |
| | | US 2011262377 A1 | 27-10-2011 |
| | | US 2015374869 A1 | 31-12-2015 |
| | | US 2017266333 A1 | 21-09-2017 |
| | | US 2018326109 A1 | 15-11-2018 |
| | | US 2022023480 A1 | 27-01-2022 |
| | | WO 2011123723 A1 | 06-10-2011 |
| US 2021205489 A1 | 08-07-2021 | KEINE | |
| CN 113041383 A | 29-06-2021 | KEINE | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20110262377 A1
- US 20210205489 A1
- DE 202021100549 U1
- US 2357846 A
- WO 2021037920 A2

**In der Beschreibung aufgeführte Nicht-Patentliteratur**

- **S. ARCTANDER.** Perfume and Flavor Chemicals. J.Selbstverlag, 1969
- **H. SURBURG ; J. PANTEN.** Common Fragrance and Flavor Materials. Wiley-VCH, 2016